# DEMANDE DE BREVET EUROPEEN

(11) **EP 1 302 197 A1**
(43) Date de publication de la demande: **16.04.2003**
(21) Numéro de dépôt: 02292445.0
(22) Date de dépôt: 04.10.2002
(51) Int. Cl.: A61K 7/42, B01F 17/42

(54) **Utilisation de copolymères amphiphiles pour stabiliser des dispersions de composés organiques insolubles filtrant le rayonnement UV**

(30) Priorité: 11.10.2001 FR 0113114
(71) Demandeur: L'OREAL, 75008 Paris (FR)
(72) Inventeur: Candau, Didier, 91570 Bièvres (FR); Aubert, Fabien, 75018 Paris (FR)
(74) Mandataire: Casalonga, Axel

(57) **Abrégé**

La demande concerne l'utilisation d'au moins un copolymère amphiphile comportant au moins une séquence hydrophile et au moins une séquence hydrophobe pour stabiliser des dispersions de composés organiques insolubles comportant au moins un groupe absorbant le rayonnement UV se présentant sous forme de particules ayant une taille moyenne comprise entre 10nm et 5µm, ainsi que les dispersions stabilisées par ces copolymères amphiphiles et des compositions cosmétiques à application topique, en particulier des compositions anti-solaires, les contenant.

## Description

La présente invention concerne l'utilisation de copolymères amphiphiles pour stabiliser des dispersions de composés organiques insolubles comportant au moins un groupe absorbant le rayonnement UV se présentant sous forme de particules ayant une taille moyenne comprise entre 10 nm et 5 µm, les dispersions obtenues, stabilisées par ces copolymères et des compositions cosmétiques les contenant, ainsi qu'un procédé de préparation de telles dispersions.

De nombreuses compositions cosmétiques destinées à la photoprotection de la peau ont été proposées à ce jour.
Ces compositions anti-solaires se présentent souvent sous la forme d'une émulsion de type huile-dans-eau ou eau-dans-huile, de gels ou de produits anhydres contenant un ou plusieurs filtres organiques et/ou minéraux, insolubles et/ou solubles, lipophiles et/ou hydrophiles, capables d'absorber sélectivement le rayonnement UV nocif. L'efficacité de ces filtres est caractérisée par leur coefficient d'extinction molaire ε. La nature et la concentration des filtres dans les compositions anti-solaires sont choisies en fonction de l'indice de protection et du profil de protection recherchés. Selon leur caractère lipophile ou hydrophile, les filtres peuvent se répartir soit dans la phase grasse, soit dans la phase aqueuse de la composition anti-solaire.
Un grand nombre de filtres organiques utilisés communément portent des substituants qui les rendent solubles dans les différents milieux cosmétiques : les chaînes de type 2-éthylhexyle, par exemple, rendent les filtres solubles dans les huiles et des groupes ionisés tels que l'acide sulfonique leur confère une certaine solubilité dans les solvants polaires et en particulier dans l'eau.
On a cependant observé que la présence de substituants sur le chromophore aboutissait à une diminution du pouvoir absorbant et il y a donc un grand intérêt à pouvoir utiliser des filtres à chromophore non substitué afin de tirer pleinement partie de leurs propriétés d'absorption.
Toutefois l'insolubilité de ces filtres dans la plupart des solvants cosmétiques rend leur manipulation difficile. On a par ailleurs constaté que, pour être pleinement efficaces, ils doivent se trouver sous forme de très fines particules parfaitement dispersées dans le milieu cosmétique.
Une des solutions pour obtenir une dispersion satisfaisante des particules de filtres dans les compositions cosmétiques consiste à réaliser d'abord une prédispersion concentrée qui sera ensuite introduite dans le milieu cosmétique.
On peut préparer de telles prédispersions de filtres organiques insolubles micronisés en broyant une dispersion de particules grossières en présence d'un ou de plusieurs agents dispersants appropriés jusqu'à la taille de particules recherchée. Ces agents dispersants sont généralement des composés possédant une activité interfaciale qui vont se localiser à l'interface solide/liquide empêchant ainsi l'agglomération et la sédimentation des particules.
La demande internationale WO 97/03643 et la demande EP 0 893 119 décrivent un procédé de broyage (micronisation) de filtres organiques insolubles en présence d'un agent dispersant choisi parmi les alkylpolyglucosides de formule CₙH₂ₙ₊₁O(C₆H₁₀O₅)ₓH où n est un nombre entier entre 8 et 16 et x vaut entre 1,4 et 1,6.
La demande internationale WO 95/22959 divulgue un procédé de micronisation de filtres organiques insolubles en présence d'un adjuvant de broyage choisi parmi les polyvinylpyrrolidones alkylées, les copolymères de vinylpyrrolidone et d'acétate de vinyle, les acylglutamates, les copolymères d'acide acrylique et de tert-octylpropénamide, les produits de condensation acide ditolyléthersulfonique-formaldhéhyde, les Carbomer®, un mélange commercial d'esters d'acide gras comprenant un tristyrylphénol éthoxylé et les phospholipides.
L'utilisation des agents dispersants et adjuvants de broyage ci-dessus pour stabiliser des dispersions de filtres organiques UV micronisés pose toutefois un certain nombre de problèmes. En effet, bien que ces agents dispersants permettent d'obtenir des prédispersions concentrées stables, la dilution des prédispersions dans le milieu cosmétique lors de la préparation des compositions anti-solaires s'accompagne généralement d'une désorption au moins partielle des agents dispersants et d'une déstabilisation de la composition cosmétique anti-solaire finale.
On a essayé de surmonter cette instabilité observée au moment de la dilution des prédispersions en augmentant la concentration des agents dispersants. Il s'est toutefois avéré qu'une telle augmentation de la concentration avait pour conséquence de nuire à la rémanence des filtres organiques insolubles sur la peau ou les cheveux, c'est-à-dire de favoriser leur élimination au contact de l'eau.

La présente invention a par conséquent pour objectif de trouver des agents dispersants permettant de stabiliser - à des concentrations relativement faibles ne nuisant pas à la rémanence des filtres - à la fois des dispersions concentrées de filtres organiques insolubles micronisés et les compositions cosmétiques obtenues par dilution de ces dispersions concentrées dans un milieu cosmétique.

La demanderesse a constaté qu'un groupe particulier de polymères ayant pour caractéristique commune de comprendre au moins une séquence de monomères hydrophiles et au moins une séquence de monomères hydrophobes, appelés "copolymères amphiphiles", permettait de stabiliser de manière satisfaisante des dispersions concentrées et des dispersions diluées de fines particules de composés organiques insolubles absorbant le rayonnement UV.

La présente invention a par conséquent pour objet l'utilisation d'au moins un copolymère amphiphile comportant au moins une séquence hydrophile et au moins une séquence hydrophobe pour stabiliser des dispersions de composés organiques insolubles comportant au moins un groupe absorbant le rayonnement UV se présentant sous forme de particules ayant une taille moyenne comprise entre 10 nm et 5 µm.

L'invention a encore pour objet un procédé de préparation de telles dispersions consistant à soumettre des particules dudit composé organique, en suspension dans un milieu liquide, à une étape de réduction de taille des particules, jusqu'à obtention d'une taille moyenne des particules comprise entre 10 nm et 5 µm, et à ajouter avant, pendant ou après l'étape de réduction de taille des particules, au moins un copolymère amphiphile comportant au moins une séquence hydrophile et au moins une séquence hydrophobe.

Elle a également pour objet des dispersions comprenant, dans un milieu liquide, au moins un composé organique insoluble comportant au moins un groupe absorbant le rayonnement UV sous forme de particules ayant une taille moyenne comprise entre 10 nm et 5 µm, et au moins un agent dispersant choisi parmi un groupe particulier de copolymères amphiphiles comportant au moins une séquence hydrophile et au moins une séquence hydrophobe, ainsi que des compositions cosmétiques à application topique, en particulier des compositions destinées à la photoprotection de la peau et/ou des cheveux, préparées par dilution de ses dispersions dans un milieu cosmétiquement acceptable.

Contrairement aux dispersions de filtres UV organiques micronisés, stabilisées par les agents dispersants de la technique antérieure (voir WO 97/03643 et WO 95/22959), les dispersions de la présente invention stabilisées par les polymères amphiphiles décrits plus en détail ci-après, restent parfaitement stables - c'est-à-dire que l'on n'observe pas de phénomènes d'agrégation, de flocculation ou de sédimentation des particules - lorsqu'on prépare à partir de ces dispersions des compositions cosmétiques par dilution dans un milieu cosmétiquement acceptable. L'effet stabilisant, constaté au moment de la dilution, est obtenu pour des quantités de polymères dispersants faibles, c'est-à-dire de l'ordre de quelques milligrammes par mètre carré de surface de particules seulement, ce qui non seulement réduit les coûts de matières premières des compositions, mais présente avant tout l'avantage de ne pas altérer la rémanence des filtres UV insolubles sur la peau et/ou les cheveux.

Les compositions cosmétiques photoprotectrices contenant des particules de filtres UV organiques insolubles, stabilisées par les polymères amphiphiles décrits ci-après se distinguent par ailleurs par un excellent pouvoir photo-protecteur et des propriétés cosmétiques satisfaisantes.

On entend par "copolymère amphiphile comportant au moins une séquence hydrophile et au moins une séquence hydrophobe" dans la présente invention, des polymères comportant au moins deux types de "séquences" ou de "blocs" de monomères, chacune des séquences (ou blocs) étant constituée d'une succession de monomères identiques. Les polymères amphiphiles de la présente peuvent avoir une structure linéaire dans laquelle les différents blocs sont enchaînés, en alternance (hydrophile/hydrophobe), les uns à la suite des autres. Ce type de polymère englobant des structures linéaires biséquencées, triséquencées ou polyséquencées, est généralement appelé dans le domaine de la chimie macromoléculaire "copolymères séquencés" ou "copolymères à blocs". La demanderesse entend cependant également englober dans la définition des copolymères amphiphiles utilisés dans la présente invention, des polymères ayant une structure ramifiée, par exemple une structure dans laquelle des séquences d'un premier type (hydrophobe ou hydrophile) sont fixées sur une chaîne principale formant un deuxième type de séquence (hydrophile ou hydrophobe). Ces polymères sont généralement appelés "copolymères de greffage" ou "copolymères à structure en peigne".
Tous ces copolymères sont connus dans la technique et peuvent être préparés selon des procédés tels que la polymérisation radicalaire suivie d'une étape de polymération-greffage, la polymérisation anionique ou cationique, la polycondensation, ou encore selon une technique récente appelée "polymérisation radicalaire contrôlée" décrite, entre autres, dans "New Method of Polymer Synthesis", Blackie Academic & Professional, Londres, 1995, volume 2, page 1, ou dans Trends Polym. Sci. 4, page 183 (1996) de C. J. Hawker, et notamment par polymérisation radicalaire par transfert d'atome décrite dans *JACS*, 117, page 5614 (1995), de Matyjasezwski et al.

Les copolymères amphiphiles comportant au moins une séquence hydrophile et au moins une séquence hydrophobe utilisés pour la stabilisation de dispersions de particules de filtres UV insolubles dans un milieu liquide sont de préférence des polymères non ioniques. En effet, étant donné que les prédispersions et les compositions cosmétiques préparées à partir de celles-ci contiennent généralement une phase aqueuse, la présence de nombreuses charges sur le polymère dispersant augmenterait excessivement sa solubilité de celui-ci dans le milieu dispersant et favoriserait par conséquent la désorption au moment de la dilution avec un milieu cosmétiquement acceptable.

On peut citer à titre d'exemples de copolymères amphiphiles préférés en tant qu'agents dispersants dans la présente invention :
- les alcools aliphatiques polyalcoxylés de formule :

   H₂ₙ₊₁Cₙ(CH₂CH₂O)ₓ(CH₂CH(CH₃)O)_{y}(CH(CH₃)CH₂O)_{z}H

   dans laquelle
   n est un nombre entier compris entre 5 et 20, de préférence entre 7 et 19,
   x est un nombre entier compris entre 2 et 20, de préférence entre 4 et 10,
   y est un nombre entier compris entre 2 et 20, de préférence entre 4 et 10,
   la somme de y + z étant un nombre entier compris entre 2 et 20, de préférence entre 2 et 12.
   Ces produits sont commercialisés sous la dénomination Synperonic®, séries LF et A, et la dénomination ATPLUS® par la société Uniquema.
- les copolymères triséquencés d'oxyde d'éthylène et d'oxyde de propylène, de formule

   HO(CH₂CH₂O)ₐ(CH₂CH(CH₃)O)_{b}(CH₂CH₂O)_{c}H

   dans laquelle a représente un nombre entier compris entre 2 et 150, b représente un nombre entier compris entre 10 et 80 et c représente un nombre entier compris 2 et 150 ;
   la balance hydrophile/lipophile de ces polymères varie en fonction de la valeur de a, b et de c.
- les produits de condensation de copolymères séquencés d'oxyde d'éthylène et d'oxyde de propylène sur l'éthylènediamine correspondant à la formule : 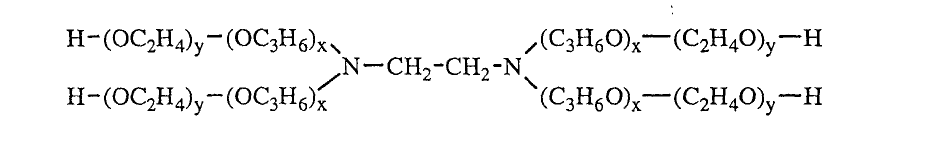 produits commercialisés par la société Uniquema,
- les copolymères séquencés de styrène et d'oxyde d'éthylène,
   Ce type de polymère est commercialisé par exemple sous la dénomination Tegomer® par la société Goldschmidt.
- les polymères diuréthanne d'alcools gras polyalcoxylés de formule : 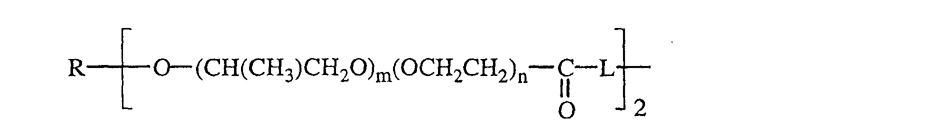 dans laquelle
   n représente un nombre compris entre 40 et 70, m représente un nombre compris entre 5 et 20, R représente un groupe alkyle en C₈₋₃₀ et L représente un groupe dérivé d'isophorone-diisocyanate ou d'hexaméthylène-diisocyanate,
   On peut citer à titre d'exemples de ces produits, le PPG-14 laureth-60 isophoryl dicarbamate (dénomination INCI) et le PPG-14 palmeth-60 hexyl dicarbamate (dénomination INCI) commercialisés respectivement sous les dénominations Elfacos® T211 et Elfacos® T212 par la société Akzo.
- les polyoléfine-succinates polyéthoxylés, et en particulier le polyisobutylène-succinate polyéthoxylé de formule 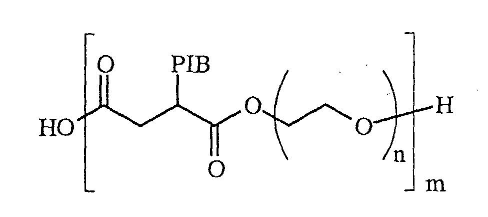 dans laquelle
   PIB représente une chaîne de polyisobutylène, n représente un nombre entier compris entre 2 et 15, de préférence entre 7 et 10, et m représente un nombre entier compris entre 2 et 15, de préférence entre 2 et 6 ;
   Ces polymères peuvent être préparés par condensation d'une polyoléfine telles que le polyisobutylène avec l'anhydride succinique selon la réaction 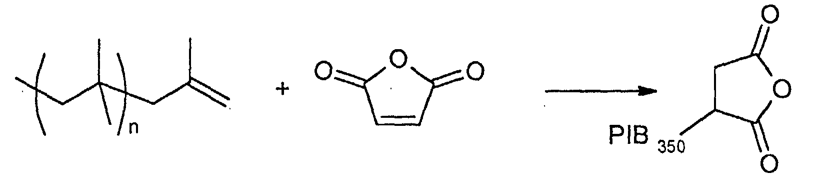 suivie d'une polyéthoxylation du polyoléfine-succinate obtenu. Ce type de produit est commercialisé par exemple sous la dénomination OS149480 par la société Lubrizol.
- les terpolymères séquencés d'acide (méth)acrylique, de (méth)acrylate d'alkyle en C₁₀₋₃₀ et de (méth)acrylate de polyéthylèneglycol.
   On peut citer comme polymères préférés de cette famille, les terpolymères d'acide acrylique, d'acrylate d'alkyle en C₁₅ et d'acrylate de polyéthylèneglycol (28 OE), commercialisés par exemple sous la dénomination Dapral® GE 202 par la société Akzo.

Les composés organiques insolubles comportant au moins un groupe absorbant le rayonnement UV utilisés dans la présente invention sont connus.

On entend par composé organique insoluble, au sens de la présente invention, un composé organique ayant une solubilité dans l'eau inférieure à 0,1 % en poids et une solubilité inférieure à 1 % en poids dans la plupart des solvants organiques comme l'huile de paraffine, les benzoates d'alcools gras et les triglycérides d'acides gras.

Les composés organiques insolubles comportant au moins un groupe absorbant le rayonnement UV peuvent être choisis notamment parmi les filtres UV organiques insolubles de type oxalanilide, triazine, benzotriazole, amide vinylique, cinnamide, benzazole, benzofuranne, arylvinylidène-cétone, amide d'acrylonitrile, sulfonamide d'acrylonitrile, carbamate d'acrylonitrile ou phénylène-bis-benzoxazinone.

Parmi les filtres UV insolubles de type oxalanilide, on peut citer ceux répondant à la formule : 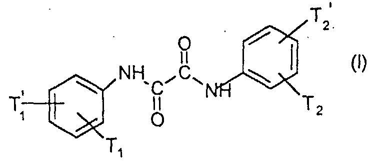 dans laquelle T₁, T₁', T₂ et T₂', représentent chacun indépendamment un radical alkyle en C₁-C₈ ou un radical alcoxy en C₁-C₈. Ces composés sont décrits dans la demande de brevet WO95/22959. A titre d'exemples, on peut citer les produits commerciaux TINUVIN® 315 et TINUVIN® 312 vendus par la Société CIBA-GEIGY correspondant respectivement aux formules :

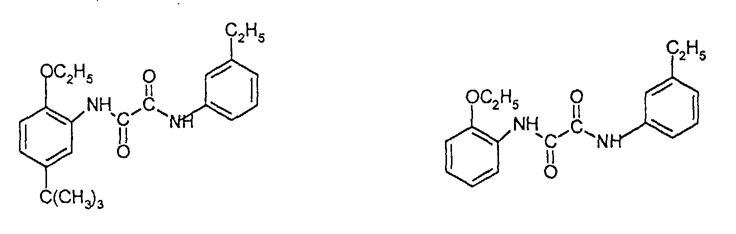

Les filtres insolubles de type triazine répondent à la formule générale suivante : 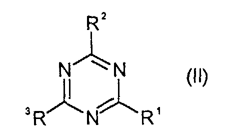 dans laquelle R₁, R₂ et R₃ représentent chacun indépendamment un groupe phényle, phénoxy ou pyrrolo non substitués ou portant chacun indépendamment un, deux ou trois substituants choisis parmi -OH, alkyle en C₁₋₁₈, alkoxy en C₁₋₁₈, carboxy(alkyle en C₁₋₁₈), cycloalkyle en C₅-C₈, méthylbenzylidène-camphre, -(CH=CR')ₙ(CO)-OR₄ où R' représente un atome d'hydrogène, un groupe cyano ou un groupe COOR₄, avec R₄ = alkyle en C₁₋₁₈ ou cinnamyle, et n vaut 0 ou 1.
Ces composés sont décrits dans WO 97/03643, GB 2286774, EP 743309, WO 98/22447, GB 2319523 et EP-A-0 790 243.

On citera plus particulièrement les composés suivants :
- la 2,4,6-tris(4'-amino benzalmalonate de diéthyle)-s-triazine,
- la 2,4,6-tris(4'-amino benzalmalonate de diisopropyle)-s-triazine,
- la 2,4,6-tris(4'-amino benzalmalonate de diméthyle)-s-triazine,
- la 2,4,6-tris(α-cyano-4-aminocinnamate d'éthyle)-s-triazine.

Parmi les filtres UV de type triazine utilisables pour la présente invention, on peut encore mentionner les dérivés insolubles de s-triazine portant des groupements benzotriazoles et/ou benzothiazoles tels que ceux décrits dans la demande WO98/25922.
Parmi ces composés, on peut citer plus particulièrement la 2,4,6-tris[(3'-benzotriazol-2-yl-2'-hydroxy-5'-methyl)-phenyl-amino]-s-triazine et la 2,4,6-tris[(3'-benzotriazol-2-yl-2'-hydroxy-5'-ter-octyl)-phényl-amino]-s-triazine.

Parmi les filtres UV organiques insolubles de type benzotriazole, on peut citer ceux de formule (III) ci-dessous, décrits par exemple dans la demande internationale WO95/22959 : 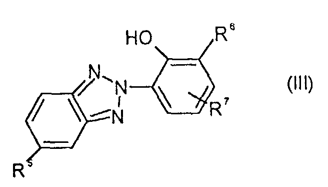 dans laquelle R₅ désigne un atome d'hydrogène ou un radical alkyle en C₁₋₁₈, R₆ et R₇, identiques ou différents, désignent chacun indépendamment un radical alkyle en C₁₋₁₈ éventuellement substitué par un groupe phényle.
A titre d'exemples de composés de formule (III), on peut citer les produits commerciaux TINUVIN® 328 , 320, 234 et 350 de la Société CIBA-GEIGY correspondant respectivement aux formules suivantes :

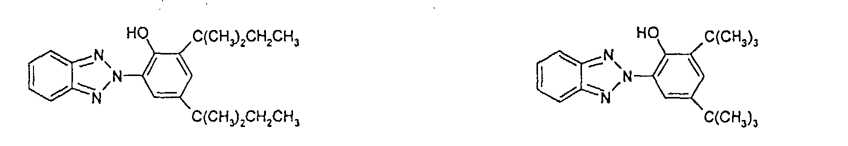

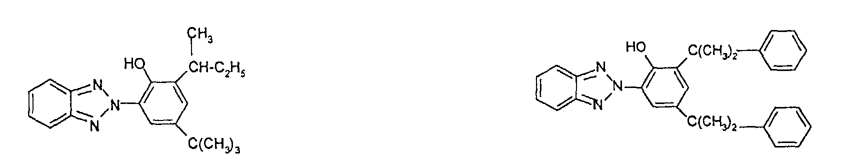

Parmi les filtres UV organiques insolubles de type benzotriazole, on peut encore citer les composés décrits dans les brevets US 5687521, US 5687521, US 5373037, US 5362881, et parmi eux en particulier le [2 ,4'-dihydroxy-3-(2H-benzotriazol-2-yl)-5-(1,1,3,3-tétraméthylbutyl)-2'-n-octoxy-5'-benzoyl]-diphénylméthane de formule : 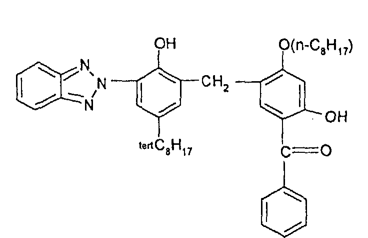 vendu sous le nom MIXXIM® PB30 par la société FAIRMOUNT CHEMICAL.

D'autres filtres UV organiques insolubles de type benzotriazole sont les dérivés de méthylène-bis-(hydroxyphénylbenzotriazole) de structure suivante: 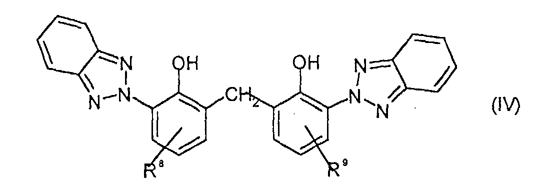 dans laquelle R₈ et R₉, identiques ou différents, représentent chacun un radical alkyle en C₁₋₁₈ pouvant être substitué par un ou plusieurs radicaux choisis parmi alkyle en C₁₋₄, cycloalkyle en C₅₋₁₂ ou aryle. Ces composés sont connus et sont décrits dans les demandes US 5237071, US 5166355, GB-A-2 303549, DE 19726184 et EP-A-893119.

Dans la formule (IV) définie ci-dessus, les groupes alkyle en C₁₋₁₈ peuvent être linéaires ou ramifiés et sont par exemple méthyle, éthyle, n-propyle, isopropyle, n-butyle, isobutyle, tert-butyle, tert-octyle, n-amyle, n-hexyle, n-heptyle, n-octyle, iso-octyle, n-nonyle, n-décyle, n-undécyle, n-dodécyle, tétradécyle, hexydécyle, ou octadécyle ; les groupes cycloalkyle en C₅₋₁₂ sont par exemple cyclopentyle, cyclohexyle, cyclooctyle ; les groupes aryle sont par exemple phényle, benzyle.

Parmi les composés de formule (IV), on préfère plus particulièrement ceux correspondant aux formules :

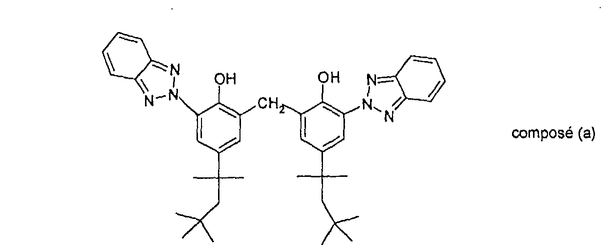

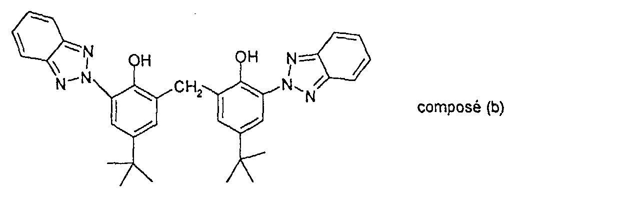

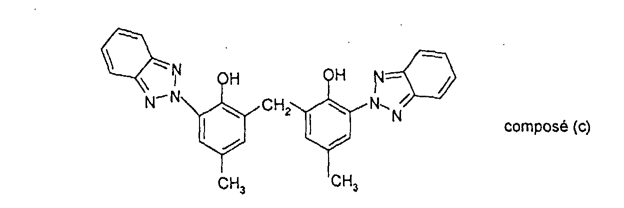

Le composé (a) 2,2'-méthylène-bis-[6-(2H-benzotriazol-2-yl)-4-(1,1,3,3-tétraméthylbutyl)phénol)] est vendu sous forme pure sous le nom MIXXIM® BB/100 par la société FAIRMOUNT CHEMICAL et sous forme micronisée sous le nom TINOSORB® M par CIBA GEIGY.
Le composé (c) 2,2'-méthylène-bis-[6-(2H-benzotriazol-2-yl)-4-(méthyl)phénol)] est vendu sous le nom MIXXIM® BB/200 par la société FAIRMOUNT CHEMICAL.

Parmi les filtres organiques insolubles du type amide vinylique, on peut citer par exemple les composés de formule (V) qui sont décrits dans la demande WO95/22959 :

T₃-(Y)ᵣ-C(=O)-C(T₄)=C(T₅)-N(T₆)(T₇) (V)

dans laquelle T₃ est un radical alkyle en C₁₋₁₈, de préférence en C₁₋₅, ou un groupe phényle éventuellement substitué par un, deux ou trois radicaux choisis parmi OH, alkyle en C₁₋₁₈, alcoxy en C₁₋₈ ou -C(=O)-OT₈ où T₈ représente un groupe alkyle en C₁₋₁₈ ; T₄, T₅, T₆ et T₇ représentent chacun indépendamment un radical alkyle en C₁₋₁₈, de préférence en C₁₋₅, ou un atome d'hydrogène ; Y représente un groupe -NH- ou un atome d'oxygène et r vaut 0 ou 1.

Parmi ces composés, on citera plus particulièrement :
la 4-octylamino-3-pentèn-2-one ;
le 3-octylamino-2-buténoate d'éthyle ;
la 3-octylamino-1-phényl-2-butèn-1-one
la 3-dodécylamino-1-phenyl-2-butèn-1-one.

Parmi les filtres organiques de type cinnamide, on peut citer les composés tels que ceux décrits dans la demande WO 95/22959 et répondant à la formule suivante : 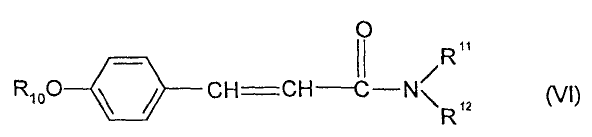 dans laquelle
R₁₀ représente un atome d'hydrogène ou un groupe alkyle en C₁₋₄ de préférence méthyle ou éthyle,
R₁₁ représente un atome d'hydrogène ou un groupe alkyle en C₁₋₄, de préférence méthyle ou éthyle,
R₁₂ représente un groupe -(CONH)ₛ-phényle où s vaut 0 ou 1 et le groupe phényle peut être substitué par un, deux ou trois groupes choisis parmi OH, alkyle en C₁₋₁₈, alcoxy en C₁₋₈ ou -C(=O)-OR₁₃ où R₁₃ est un alkyle en C₁₋₁₈, et plus préférentiellement R₁₂ représente un groupe phényle, 4-méthoxyphényle ou phénylaminocarbonyle.

On peut également citer les dimères bis-[cinnamides α,β disubstitués] , décrits par exemple dans le brevet US 5 888 481, de structure : 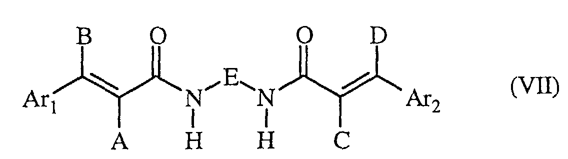 dans laquelle
Ar₁ et Ar₂, identiques ou différents, représentent chacun un radical phényle, un hétérocycle aromatique, un groupe à noyau phényle condensé ou un groupe à hétérocycle aromatique condensé, et peuvent porter un ou plusieurs substituants, identiques ou différents,
B et D, différents d'un atome d'hydrogène, représentent chacun indépendamment un radical organique,
A et C représentent chacun indépendamment un radical organique, et
E représente un radical organique divalent,
à l'exclusion des composés pour lesquels Ar₁ et Ar₂ représentent tous les deux un groupe phényle portant un substituant -OR où R représente un atome d'hydrogène ou un radical organique, A et C représentent tous les deux un groupe cyano, B et D représentent tous les deux un groupe alkyle ou alcényle en C₁₋₃₅, et E représente un radical organique divalent,
et en particulier le composé de structure :

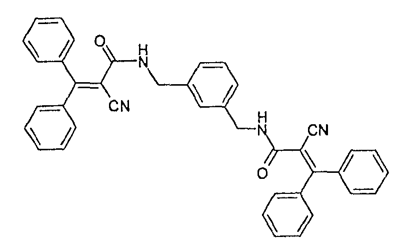

Parmi les filtres organiques insolubles du type benzazole, on peut citer ceux répondant à l'une des formules suivantes : 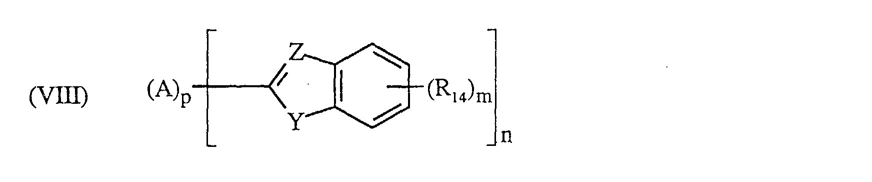 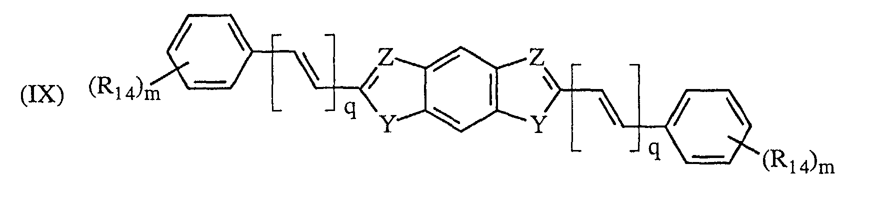 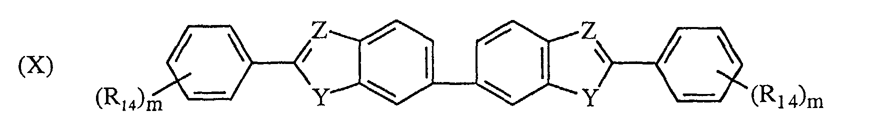 dans lesquelles
chacun des symboles Y représente indépendamment un atome d'oxygène ou de soufre ou un groupe NR₁₅,
chacun des symboles Z représente indépendamment un atome d'azote ou un groupe CH,
chacun des symboles R₁₄ représente indépendamment un groupe OH, un atome d'halogène, un groupe alkyle en C₁₋₈, linéaire ou ramifié, contenant éventuellement un atome de silicium, ou un groupe alcoxy en C₁₋₈, linéaire ou ramifié,
chacun des nombres m vaut indépendamment 0, 1 ou 2,
n représente un nombre entier compris entre 1 et 4 inclus,
p est égal à 0 ou 1,
chacun des nombres q est égal indépendamment à 0 ou 1,
chacun des symboles R₁₅ représente indépendamment un atome d'hydrogène, un groupe benzyle ou alkyle en C₁₋₈, linéaire ou ramifié, contenant éventuellement un atome de silicium,

A représente un radical de valence n choisi parmi ceux de formules 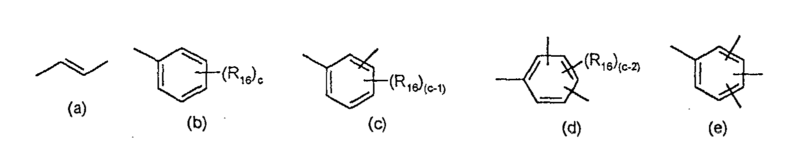 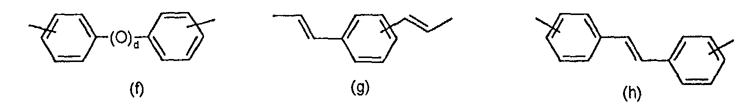 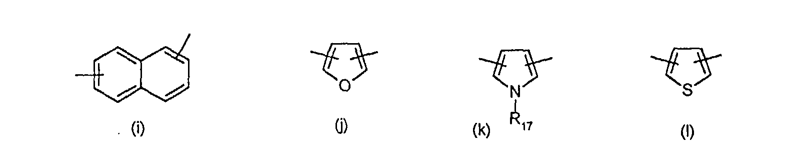 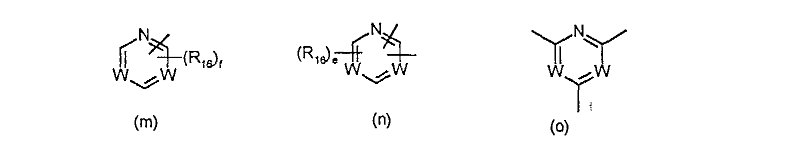
dans lesquelles chacun des symboles R₁₆ représente indépendamment un atome d'halogène ou un groupe alkyle ou alcoxy en C₁₋₄, linéaire ou ramifié, ou hydroxy,
R₁₇ représente un atome d'hydrogène ou un groupe alkyle en C₁₋₄, linéaire ou ramifié,
c = 0 - 4, d = 0 - 3, e = 0 ou 1 et f = 0 - 2.

Ces composés sont notamment décrits dans les brevets DE 676 103 et CH 350763, le brevet US 5501850, le brevet US 5961960, la demande de brevet EP 0669323, le brevet US 5518713, le brevet US 2463264, l'article du *J. Am. Chem. Soc.,* 79, 5706 - 5708, 1957, l'article publié dans *J. Am. Chem. Soc.,* 82, 609 - 611, 1960, la demande de brevet EP 0921126 et la demande de brevet EP 0712855.

A titre d'exemples de composés préférés de formule (VIII) de la famille des 2-arylbenzazoles, on peut mentionner le 2-benzoxazol-2-yl-4-méthylphénol, le 2-(1H-benzimidazol-2-yl)-4-méthoxyphénol ou le 2-benzothiazol-2-ylphénol, ces composés pouvant être préparés par exemple selon les procédés décrits dans le brevet CH 350 763.

A titre d'exemples de composés préférés de formule (VIII) de la famille des benzimidazolylbenzazoles, on citera le 2,2'-bis-benzimidazole, le 5,5',6,6'-tétraméthyl-2,2'-bis-benzimidazole, le 5,5'-diméthyl-2,2'-bis-benzimidazole, le 6-méthoxy-2,2'-bis-benzimidazole, le 2-(1H-benzimidazol-2-yl)-benzothiazole, le 2-(1H-benzimidazol-2-yl)-benzoxazole et le N,N'-diméthyl-2,2'-bis-benzimidazole, ces composés pouvant être préparés selon les modes opératoires décrits dans les brevets US 5961960 et US 2463264.

A titre d'exemples de composés préférés de formule (VIII) de la famille des phénylène-benzazoles, on citera le 1,4-phénylène-bis-(2-benzoxazolyle), le 1,4-phénylène-bis-(2-benzimidazolyle), le 1,3-phénylène-bis-(2-benzoxazolyle), le 1,2-phénylène-bis-(2-benzoxazolyle), le 1,2-phénylène-bis-(benzimidazolyle), le 1,4-phénylène-bis-(N-2-éthylhexyl-2-benzimidazolyle) et le 1,4-phénylène-bis-(N-triméthylsilylméthyl-2-benzimidazolyle), ces composés pouvant être préparés selon les modes opératoires décrits dans le brevet US 2 463 264 et dans les publications *J. Am.Chem. Soc.,* 82, 609 (1960) et *J. Am. Chem. Soc*., 79, 5706 - 5708 (1957).

A titre d'exemples de composés préférés de formule (VIII) de la famille des benzofuranyl-benzoxazoles, on citera le 2-(2-benzofuranyl)-benzoxazole, le 2-(benzofuranyl)-5-méthylbenzoxazole et le 2-(3-méthyl-2-benzofuranyle)-benzoxazole, ces composés pouvant être préparés selon les modes opératoires décrits dans le brevet US 5518713.

Comme composés préférés de formule (IX), on peut citer par exemple le 2,6-diphényl-1,7-dihydro-benzo[1,2-d;4,5-d']-diimidazole correspondant à la formule 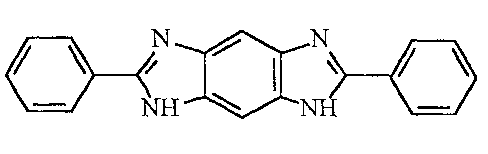 ou le 2,6-distyryl-1,7-dihydro-benzo[1,2-d; 4,5-d']-diimidazole ou encore le 2,6-di(p-tert-butylstyryl)-1,7-dihydrobenzo[1,2-d ; 4,5-d']-diimidazole, qui peuvent être préparés selon les procédés décrits dans la demande EP 0 669 323.

Comme composé préféré de formule (X), on peut citer le 5,5'-bis-[(phényl-2)-benzimidazole] de formule : 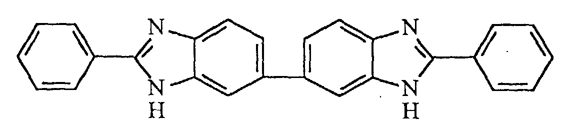 dont la préparation est décrite dans *J. Chim. Phys.,* 64, 1602 (1967).

Parmi ces composés organiques insolubles filtrant le rayonnement UV, on préfère tout particulièrement le 2-(1H-benzimidazol-2-yl)benzoxazole, le 6-méthoxy-2,2'-bis-benzimidazole, le 2-(1H-benzimidazol-2-yl)-benzothiazole, le 1,4-phénylène-bis-(2-benzoxazolyle), le 1,4-phénylène-bis-(2-benzimidazolyle), le 1,3-phénylène-bis-(2-benzoxazolyle), le 1,2-phénylène-bis-(2-benzoxazolyle), le 1,2-phénylène-bis-(2-benzimidazolyle) et le 1,4-phénylène-bis-(N-triméthylsilylméthyl-2-benzimidazolyle).

Une autre famille de filtres insolubles est celle des arylvinylènecétones choisies parmi celles correspondant à l'une des formules (XI) et (XII) suivantes : 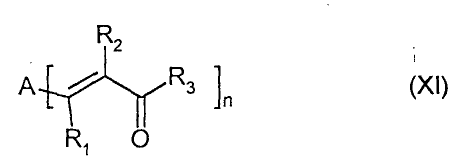 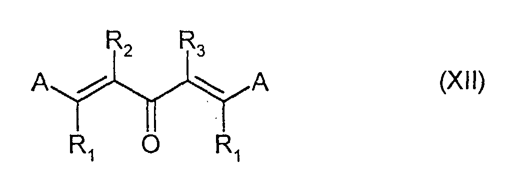 dans lesquelles :
n = 1 ou 2,
A, dans la formule (XI) lorsque n = 1 ou dans la formule (XII), est un radical aryle choisi parmi les formules (a) à (d) suivantes, ou dans la formule (XI) lorsque n=2, est un radical choisi parmi les formules (e) à (h) suivantes : 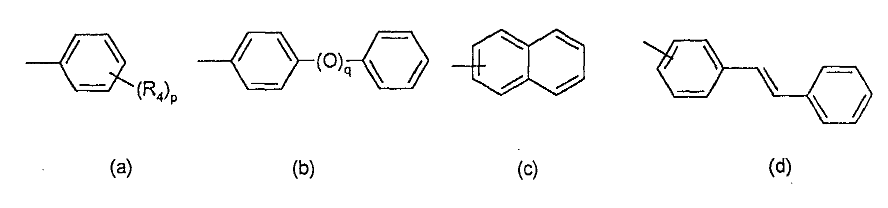 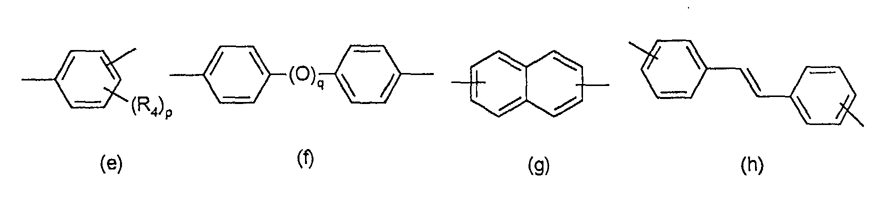
dans lesquelles :
chacun des symboles R₄ représente indépendamment un groupe OH, un atome d'halogène, un groupe alkyle en C₁₋₆, linéaire ou ramifié et contenant éventuellement un atome de silicium, un groupe alcoxy en C₁₋₆, linéaire ou ramifié et contenant éventuellement un atome de silicium, un groupe alcoxycarbonyle en C₁₋₅, linéaire ou ramifié, ou un groupe alkylsulfonamide en C₁₋₆, linéaire ou ramifié et contenant éventuellement un atome de silicium ou une fonction aminoacide,
p représente un nombre entier compris entre 0 et 4 inclus,
q représente 0 ou 1,
R₁ représente l'hydrogène ou un groupe OH,
R₂ représente l'hydrogène, un groupe alkyle en C₁₋₆, linéaire ou ramifié et contenant éventuellement un atome de silicium, un groupe cyano, un groupe alkylsulfonyle en C₁₋₆, un groupe phénylsulfonyle,
R₃ représente un groupe alkyle en C₁₋₆, linéaire ou ramifié et contenant éventuellement un atome de silicium ou un groupe phényle pouvant former un bicycle et éventuellement substitué par un ou deux radicaux R₄, ou R₂ et R₃ forment ensemble un reste hydrocarboné en C₂₋₁₀ monocyclique, bicyclique ou tricylique, éventuellement interrompu par un ou des atomes d'azote, de soufre et d'oxygène et pouvant contenir un autre carbonyle, et éventuellement substitué par un groupe alkylsulfonamide en C₁-C₈, linéaire ou ramifié, et contenant éventuellement un atome de silicium ou une fonction aminoacide ; à condition que lorsque n=1, R₂ et R₃ ne forment pas un noyau camphre.

A titre d'exemples de composés de formule (XI) dans laquelle n=1, insolubles, filtrant le rayonnement UV, on peut mentionner les familles suivantes :
- Styryl Cétone (Kao JP 04 134 042) telle que la 1-(3,4-diméthoxyphényl)-4,4-diméthyl-pent-1-èn-3-one : 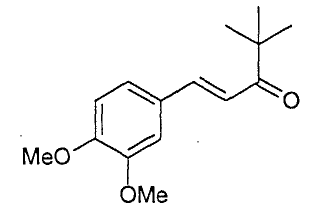
- Benzylidène Cinéole (E. Mariani et al, 16^{th} IFSCC Congress, New York (1990)) tel la 1,3,3-triméthyl-5-(4-méthoxy-benzylidène)-2-oxa-bicyclo[2.2.2]octan-6-one : 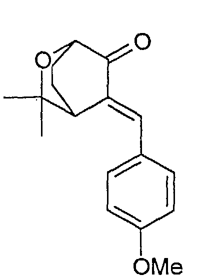
- Benzylidène Chromanone (Kao JP 04 134 043) telle que la 3-(4-méthoxy-benzylidène)-2,3,4a,8a-tétrahydro-chromèn-4-one : 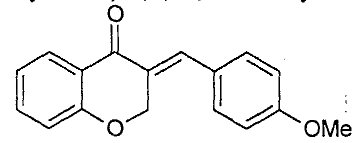
- Benzylidène Thiochromanone (Kao JP 04 134 043) telle que la 3-(4-méthoxy-benzylidène)-2,3,4a,8a-tétrahydro-chromèn-4-thione : 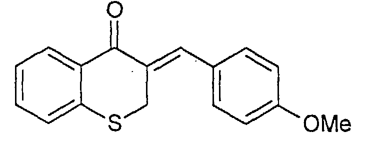
- Benzylidène Quinuclidinone (Merck EP 0 576 974) telle que la 4-méthoxy benzylidène-1-aza-bicyclo[2.2.2]octan-3-one: 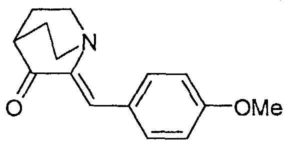
- Benzylidène Cycloalcanone (Henkel FR 2 395 023) telle que les 2-(4-méthoxy-benzylidène)-cyclopentanone et 2-(4-méthoxy-benzylidène)-cyclohexanone : 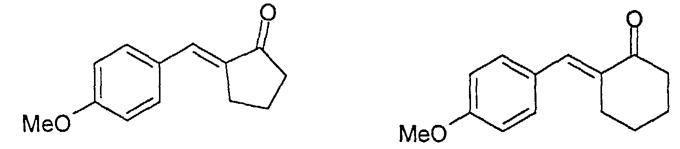
- Benzylidène Hydantoïne (Ajinomoto JP 01 158 090) telle que la 5-(3,4-diméthoxy-benzylidène)-imidazolidine-2,4-dione : 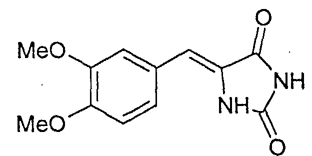
- Benzylidène Indanone (Kao JP 04 134 043) telle que la 2-(4-méthoxy-benzylidène)-indan-1-one : 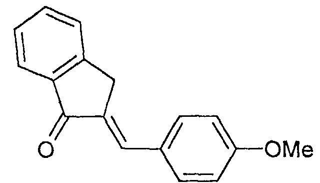
- Benzylidène Tétralone (Kao JP 04 134 043) telle que la 2-(4-méthoxy-benzylidène)-3,4-dihydro-2H-naphthalen-1-one : 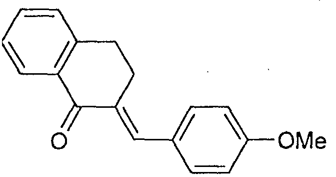
- Benzylidène Furanone (L'Oréal EP 0 390 683) telle que la 4-(4-méthoxy-benzylidène)-2,2,5,5-tétraméthyl-dihydro-furan-3-one : 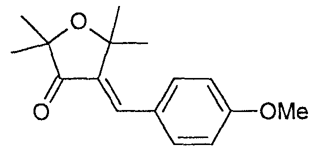
- Benzylidène Benzofuranone (Kao JP 04 134 041) telle que la 2-benzylidène-benzofuran-3-one : 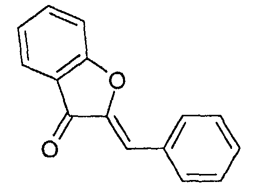
- Benzylidène Indanedione telle que la 2-(3,5-di-tert-butyl-4-hydroxy-benzylidène)-indan-1,3-dione : 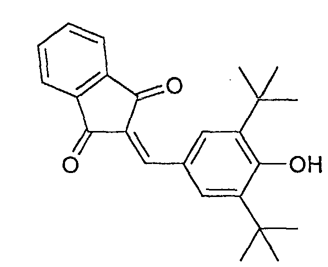
- Benzylidène Benzothiofuranone (Kao JP 04,134,043) telle que la 2-benzylidène-benzo[b]thiophen-3-one : 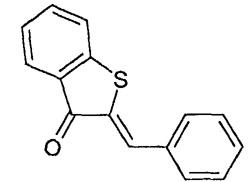
- Benzylidène Barbiturique tel que la 5-(4-méthoxy-benzylidène)-1,3-diméthyl-pyrimidine-2,4,6-trione : 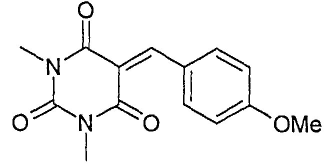
- Benzylidène Pyrazolone telle que la 4-(4-méthoxy-benzylidène)-5-méthyl-2-phényl-2,4-dihydro-pyrazol-3-one : 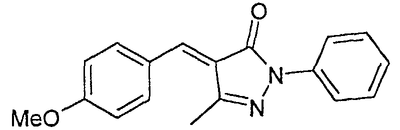
- Benzylidène Imidazolone telle que la 5-(4-méthoxy-benzylidène)-2-phényl-3,5-dihydro-imidazol-4-one : 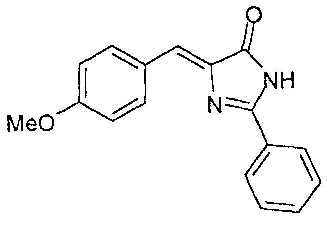
- Chalcone telle que la 1-(2-hydroxy-4-méthoxy-phényl)-3-phényl-propènone : 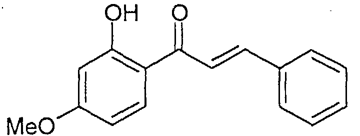
- Benzylidène One (forme tautomère filtrante des dibenzoylméthanes; L'Oréal FR 2 506 156) telle que la 3-hydroxy-1-(2-hydroxy-4-méthoxy-phényl)-3 -phényl-propènone :

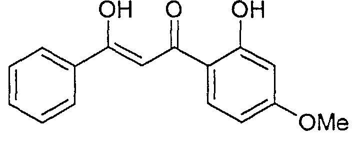

A titre d'exemples de composés de formule (XI) dans laquelle n=2 insolubles, filtrant le rayonnement UV, on peut mentionner les familles suivantes :
- Phénylène bis méthylidène-nor-camphre (Merck EP 0 693 471) tel que la 1,4-phénylène-bis-{3-méthylidène-bicyclo[2.2.1]heptan-2-one} : 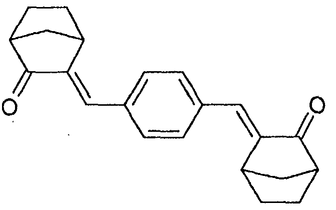
- Phénylène bis méthylidène camphre (L'Oréal FR 2 528 420) telle que la 1,4-phénylène-bis-{3-méthylidène-1,7,7-triméthyl-bicyclo [2.2.1]heptan-2-one} : 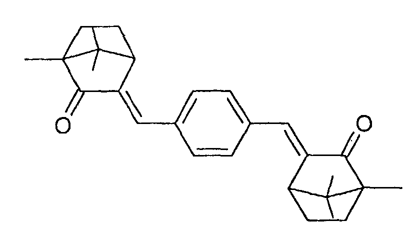 ou la 1,3-phénylène-bis-{3-méthylidène-1,7,7-triméthyl-bicyclo [2.2.1]heptan-2-one} : 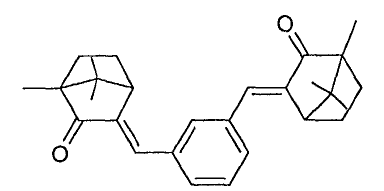
- Phénylène bis méthylidène camphre sulfonamide (L'Oréal FR 2 529 887) tel le 1,4-phénylène-bis-3,3'-méthylidène camphre-10,10'-sulfonamide d'éthyle ou de 2-éthylhexyle : 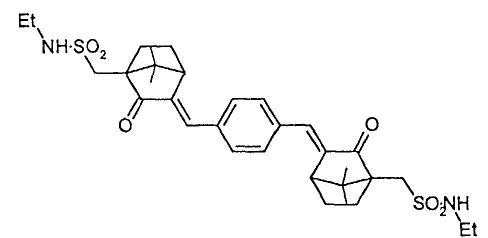 ou 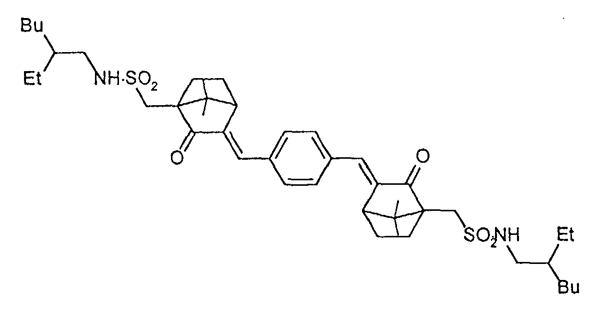
- Phénylène bis méthylidène cinéole (E. Mariani et al, 16^{th} IFSCC Congress, New York (1990)) tel la 1,4-phénylène-bis-{5-méthylidène-3,3-diméthyl-2-oxa-bicyclo[2.2.2]octan-6-one} : 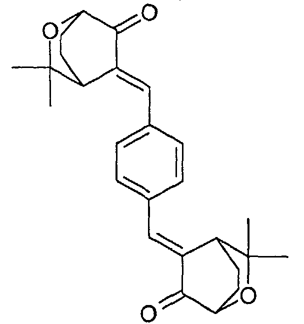
- Phénylène bis méthylidène cétotricyclodécane (Merck EP 0 694 521) tel la 1,4-phénylène-bis-(octahydro-4,7-méthano-6-indèn-5-one) : 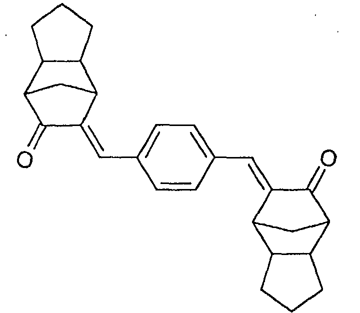
- Phénylène bis alkylène cétone (Kao JP 04 134 041) telle que la 1,4-phénylène-bis-(4,4-diméthyl-pent-1-èn-3-one) : 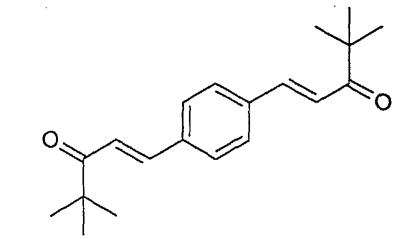
- Phénylène bis méthylidène furanone (L'Oréal FR 2 638 354) telle que la 1,4-phénylène-bis-(4-méthylidène-2,2,5,5-tétraméthyldihydrofuran-3-one) : 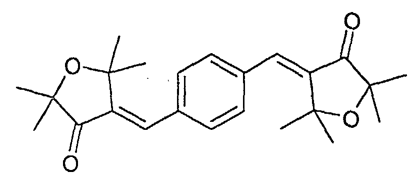
- Phénylène bis méthylidène quinuclidinone (Merck EP 0 714 880) telle que la 1,4-phénylène-bis-{2-méthylidène-1-aza-bicyclo [2.2.2]octan-3-one}:

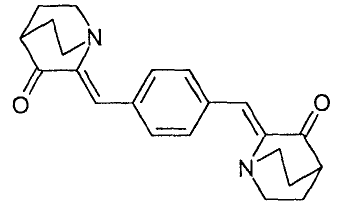

A titre de composés de formule (XII), on peut mentionner les familles suivantes :
- bis benzylidène cycloalcanone telle que la 2,5-dibenzylidène-cyclopentanone: 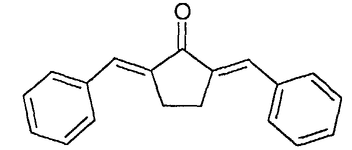
- gamma pyrone (Kao JP 04 290 882) telle que la 2,6-bis-(3,4-diméthoxy-phényl)-pyran-4-one :

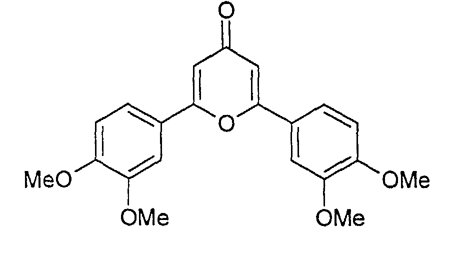

Une autre famille de filtres insolubles utilisables dans la présente invention sont les dérivés amides, sulfonamides et carbamate d'acrylonitrile correspondant à la formule suivante 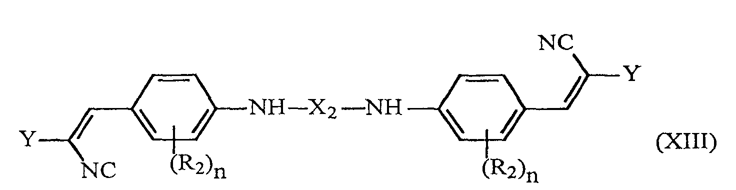 dans laquelle
X₂ représente un radical divalent de formule -(C=O)-R'₃-(C=O)-,
-SO₂-R"₃-SO₂- ou -(C=O)-O-R"₃-O-(C=O)-,
Y représente un radical -(C=O)-R₄ ou -SO₂R₅,
R₂ représente un groupe alkyle en C₁₋₈, linéaire ou ramifié,
n vaut 0, 1 ou 2,
R'₃ représente une simple liaison ou R"₃
R"₃ représente un radical divalent alkylène en C₁₋₃₀ ou alcénylène en C₃₋₃₀, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium,.
R₄ représente un radical -OR₆ ou -NHR₆,
R₅ représente un radical alkyle en C₁₋₃₀, linéaire ou ramifié, ou un noyau phényle pouvant être substitué par des radicaux alkyle ou alcoxy en C₁₋₄,
R₆ représente un radical alkyle en C₁₋₃₀ ou alcényle en C₃₋₃₀, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium.

Bien que dans la formule (XIII) ci-dessus, seuls soient représentés les isomères dans lesquelles le substituant cyano est en position cis par rapport au substituant para-amino-phényle, cette formule doit être comprise comme englobant également les isomères trans correspondants pour chacune des deux double liaisons et de façon indépendante, les substituants cyano et para-amino-phényle peuvent être en configuration cis ou trans l'un par rapport à l'autre.

Une autre famille de filtres organiques insolubles utilisables selon la présente invention est formée par les dérivés de phénylène-bis-benzoxazinone de formule 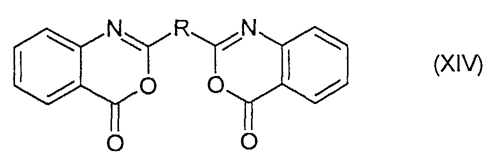 dans laquelle R représente un reste aromatique divalent choisi parmi les formules (e) à (h) suivantes : 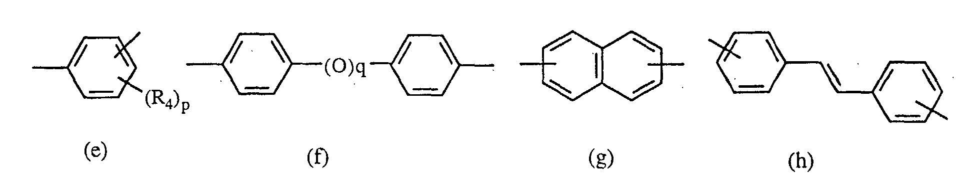 dans lesquelles :
chacun des symboles R₄ représente indépendamment un groupe OH, un atome d'halogène, un groupe alkyle en C₁₋₆, linéaire ou ramifié et contenant éventuellement un atome de silicium, un groupe alcoxy en C₁₋₆, linéaire ou ramifié et contenant éventuellement un atome de silicium, un groupe alcoxycarbonyle en C₁₋₅, linéaire ou ramifié, ou un groupe alkylsulfonamide en C₁₋₆, linéaire ou ramifié et contenant éventuellement un atome de silicium ou une fonction aminoacide,
p représente un nombre entier compris entre 0 et 4 inclus,
q représente 0 ou 1.

A titre d'exemples de composés de formule (XIV), insolubles, filtrant le rayonnement UV, on peut mentionner les dérivés suivants :
2,2'-p-phénylène bis(3,1-benzoxazin-4-one), produit commercial CYASORB® UV-3638 de la société CYTEC,
2,2'-(4,4'-biphénylène) bis (3,1-benzoxazin-4-one),
2,2'-(2,6-naphthylène) bis (3,1-benzoxazin-4-one).

Une autre famille particulière de filtres organiques insolubles sont les sels de métaux polyvalents (par exemple Ca²⁺, Zn²⁺, Mg ²⁺, Ba²⁺, Al³⁺ ou Zr⁴⁺) de filtres organiques sulfonés ou carboxylés tels que les sels de métaux polyvalents de dérivés sulfonés de benzylidène-camphre tels que ceux décrits dans la demande FR-A 2 639 347, les sels de métaux polyvalents de dérivés sulfonés de benzimidazole tels que ceux décrits dans la demande EP-A-893 119, et les sels de métaux polyvalents de dérivés d'acide cinnamique tels que ceux décrits dans la demande JP-87 166 517.

On peut également citer les complexes de métaux ou d'ammonium ou d'ammonium substitué de filtres organiques UV-A et/ou UV-B tels que ceux décrits dans les demandes de brevet WO93/10753, WO93/11095 et WO95/05150.

Les dispersions de filtres UV organiques insolubles que l'on se propose de stabiliser à l'aide des copolymères amphiphiles décrits ci-dessus sont de préférence des dispersions aqueuses, c'est-à-dire que l'eau constitue la totalité ou la plus grande partie du milieu de dispersion liquide.
Elles contiennent de préférence de 5 % à 70 % en poids, en particulier de 30 % à 50 % en poids de particules de composé organique insoluble comportant au moins un groupe absorbant le rayonnement UV.

Comme indiqué précédemment, la taille moyenne des particules de composé organique insoluble comportant au moins un groupe absorbant le rayonnement UV est comprise entre 10 nm et 5 µm.
La taille moyenne des particules de composé organique insoluble comportant au moins un groupe absorbant le rayonnement UV est de préférence comprise entre 10 nm et 2 µm, en particulier entre 20 nm et 1,5 µm, et idéalement entre 30 nm et 1,0 µm.
D'une manière générale, la taille moyenne des particules correspondra au diamètre moyen de la distribution en nombre.
La taille moyenne des particules peut être déterminée par toute méthode classique telle que les méthodes optiques (diffusion quasi-élastique ou diffusion laser), les méthodes par centrifugation ou les méthodes de visualisation microscopique et analyse d'image.

La quantité de copolymère amphiphile nécessaire pour obtenir des propriétés de stabilité et de rémanence satisfaisantes dépend, entre autres, de la nature du copolymère amphiphile et de la nature, de la concentration, de la taille et de la surface spécifique des particules de filtres UV organiques insolubles à stabiliser.
Il s'est avéré qu'il était possible de stabiliser de manière satisfaisante les dispersions de filtres UV organiques insolubles de la présente invention en utilisant une quantité de copolymère amphiphile comportant au moins une séquence hydrophile et au moins une séquence hydrophobe comprise entre 1 et 10 mg/m², de préférence entre 1 et 5 mg/m², de surface de particules de composé organique insoluble comportant au moins un groupe absorbant le rayonnement UV.

La présente invention a également pour objet un procédé de préparation de dispersions de composés organiques insolubles comportant au moins un groupe absorbant le rayonnement UV. Ce procédé consiste à soumettre des particules dudit composé organique, en suspension dans un milieu liquide, à une étape de réduction de taille des particules jusqu'à obtention d'une taille moyenne comprise entre 10 nm et 5 µm, et à ajouter avant, pendant ou après l'étape de réduction de taille, au moins un copolymère amphiphile comportant au moins une séquence hydrophile et au moins une séquence hydrophobe tels que ceux décrits ci-dessus.

L'étape de réduction de taille des particules du procédé de fabrication de dispersions de la présente invention peut être mise en oeuvre par tout moyen approprié, tel que broyage à sec ou en milieu solvant, tamisage, atomisation, micronisation, pulvérisation ou tout procédé chimique tel que précipitation par émulsion ou dilution.
Selon un mode de réalisation préféré du procédé de la présente invention, l'étape de réduction de taille des particules est un broyage dans un milieu aqueux.

Bien qu'il soit possible d'ajouter le copolymère amphiphile seulement après l'étape de broyage, un mode de réalisation préféré comprend l'addition du copolymère amphiphile avant broyage dans le prémix, c'est-à-dire dans la suspension de particules grossières dans un milieu liquide, ou bien pendant l'étape de broyage.

Un exemple de procédé de réduction de taille des particules par broyage de filtres organiques insolubles est décrit dans les documents WO 95/22959 et WO 97/03643.
L'appareil de broyage utilisé selon ces documents peut être un broyeur à jet, à billes, à vibration ou à marteau et de préférence un broyeur à haute vitesse d'agitation ou un broyeur à impact et plus particulièrement un broyeur à billes rotatives, un broyeur vibrant, un broyeur à tube ou un broyeur à tige.

L'invention a également pour objet des dispersions de particules de composés organiques insolubles comportant au moins un groupe absorbant le rayonnement UV préparées selon le procédé décrit ci-dessus, contenant un agent dispersant choisi dans un groupe particulier de copolymères amphiphiles comportant au moins une séquence hydrophile et au moins une séquence hydrophobe, ainsi que des compositions cosmétiques à application topique, en particulier des compositions destinées à la photoprotection de la peau et/ou des cheveux, préparées par dilution de ses dispersions dans un milieu cosmétiquement acceptable.
Le groupe particulier de copolymères amphiphiles englobe :
- les alcools aliphatiques polyalcoxylés de formule :

   H₂ₙ₊₁Cₙ(CH₂CH₂O)ₓ(CH₂CH(CH₃)O)_{y}(CH(CH₃)CH₂O)_{z}H

   dans laquelle
   n est un nombre entier compris entre 5 et 20, de préférence entre 7 et 19,
   x est un nombre entier compris entre 2 et 20, de préférence entre 4 et 10,
   y est un nombre entier compris entre 2 et 20, de préférence entre 4 et 10,
   la somme de y + z étant un nombre entier compris entre 2 et 20, de préférence entre 2 et 20 ;
- les copolymères triséquencés d'oxyde d'éthylène et d'oxyde de propylène, de formule

   HO(CH₂CH₂O)ₐ(CH₂CH(CH₃)O)_{b}(CH₂CH₂O)_{c}H

   dans laquelle a représente un nombre entier compris entre 2 et 150, b représente un nombre entier compris entre 10 et 80 et c représente un nombre entier compris 2 et 150 ;
- les produits de condensation de copolymères séquencés d'oxyde d'éthylène et d'oxyde de propylène sur l'éthylènediamine correspondant à la formule : 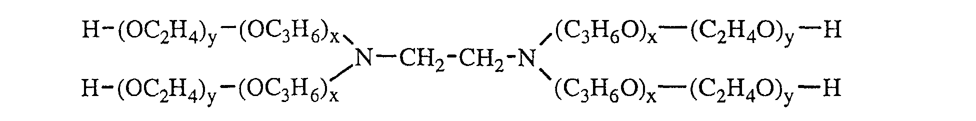
- les copolymères séquencés de styrène et d'oxyde d'éthylène,
- les polyoléfine-succinates polyéthoxylés, et en particulier le polyisobutylène-succinate polyéthoxylé de formule 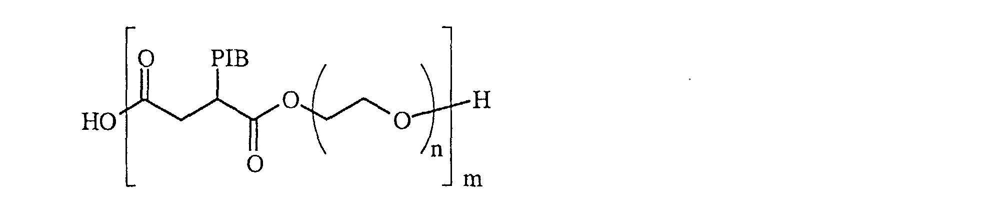 dans laquelle
   PIB représente une chaîne de polyisobutylène, n représente un nombre entier compris entre 2 et 15, de préférence entre 7 et 10, et m représente un nombre entier compris entre 2 et 15, de préférence entre 2 et 6 ;
- les terpolymères séquencés d'acide (méth)acrylique, de (méth)acrylate d'alkyle en C₁₀₋₃₀ et de (méth)acrylate de polyéthylèneglycol.

Les composés organiques insolubles comportant au moins un groupe absorbant le rayonnement UV, la taille moyenne des particules de ces filtres ainsi que la quantité de copolymère amphiphile utilisée pour les stabiliser sont les mêmes que ceux spécifiés ci-dessus.

Les compositions cosmétiques de la présente invention, obtenues par dilution dans un milieu cosmétiquement acceptable, contiennent de préférence de 0,1 à 15 % en poids, et en particulier de 0,2 à 10 % en poids de particules de composé organique insoluble comportant au moins un groupe absorbant le rayonnement UV.

Les compositions cosmétiques de la présente invention peuvent contenir en outre un ou plusieurs filtres solaires organiques hydrosolubles ou liposolubles, actifs dans le domaine des UV-A et/ou UV-B.
Ces filtres solaires hydrosolubles ou liposolubles peuvent être notamment choisis parmi les dérivés cinnamiques, les dérivés de dibenzoylméthane, les dérivés salicyliques, les dérivés du benzylidènecamphre, les dérivés solubles de triazine tels que ceux décrits dans les brevets ou demandes de brevet US 4 367 390, EP 0 863 145, EP 0 517 104, EP 0 570 838, EP 0 796 851, EP 0 775 698, EP 0 878 469, EP 0 933 376, EP 0 507 691, EP 0 507 692, EP 0 790 243, EP 0 944 624 et US 4 724 137 ; les dérivés de la benzophénone ; les dérivés de β,β'-diphénylacrylate ; les dérivés de phénylbenzimidazole ; les dérivés anthraniliques ; les dérivés d'imidazolines ; les dérivés de l'acide p-aminobenzoïque, les polymères hydrocarbonés filtres et les silicones filtres tels que ceux décrits notamment dans la demande WO 93/04665 ; les dimères dérivés d'α-alkyl styrène tels que décrits dans la demande DE 198 55 649 ; ainsi que les mélanges de ces filtres.
On peut citer à titre d'exemples de tels filtres solaires additionnels hydrosolubles ou liposolubles, actifs dans l'UV-A et/ou l'UV-B, les composés suivants désignés par leur nom INCI, ainsi que leur mélanges :

### dérivés d'acide para-aminobenzoïque :

- PABA
- Ethyl PABA
- Ethyl Dihydroxypropyl PABA
- Ethylhexyl Dimethyl PABA vendu notamment sous le nom commercial ESCALOL 507 par ISP,
- Glyceryl PABA,
- PEG-25 PABA vendu sous le nom commercial UVINUL P25 par BASF,

### dérivés salicyliques :

- Homosalate vendu sous le nom commercial EUSOLEX HMS par RONA/EM INDUSTRIES,
- Ethylhexyl Salicylate vendu sous le nom commercial NEO HELIOPAN OS par HAARMANN ET REIMER,
- Dipropyleneglycol Salicylate vendu sous le nom commercial DIPSAL par SCHER,
- TEA Salicylate vendu sous le nom commercial NEO HELIOPAN TS par HAARMANN ET REIMER,

### dérivés de dibenzoylméthane :

- Butyl Methoxydibenzoylmethane vendu notamment sous le nom commercial PARSOL 1789 par HOFFMANN LAROCHE,
- Isopropyl Dibenzoylmethane,

### dérivés cinnamiques :

- Ethylhexyl Methoxycinnamate vendu notamment sous le nom commercial PARSOL MCX par HOFFMANN LAROCHE,
- Isopropyl Methoxycinnamate
- Isoamyl Methoxycinnamate vendu sous le nom commercial NEO HELIOPAN E 1000 par HAARMANN ET REIMER,
- Cinoxate,
- DEA Methoxycinnamate,
- Diisopropyl Methylcinnamate,
- Glyceryl Ethylhexanoate Dimethoxycinnamate

### dérivés de β,β-diphénylacrylate :

- Octocrylene vendu notamment sous le nom commercial UVINUL-539 par BASF

### dérivés de benzophénone :

- Benzophenone-1 vendue sous le nom commercial UVINUL 400 par BASF,
- Benzophenone-2 vendue sous le nom commercial UVINUL D-50 par BASF,
- Benzophenone-3 ou Oxybenzone vendue sous le nom commercial UVINUL M-40 par BASF,
- Benzophenone-4 vendue sous le nom commercial UVINUL MS-40 par BASF,
- Benzophenone-5,
- Benzophenone-6 vendue sous le nom commercial HELISORB 11 par NORQUAY,
- Benzophénone-8 vendue sous le nom commercial SPECTRA-SORB UV-24 par AMERICAN CYANAMID
- Benzophenone-9 vendue sous le nom commercial UVINUL DS-49 par BASF,
- Benzophénone-12

### dérivés du benzylidène camphre :

- Benzylidene Camphor Sulfonic Acid fabriqué sous le nom MEXORYL SL par CHIMEX,
- Camphor Benzalkonium Methosulfate fabriqué sous le nom MEXORYL SO par CHIMEX,
- Terephthalylidene Dicamphor Sulfonic Acid fabriqué sous le nom MEXORYL SX par CHIMEX,
- Polyacrylamidomethyl Benzylidene Camphor fabriqué sous le nom MEXORYL SW par CHIMEX,

### dérivés de phénylbenzimidazole :

- Phenylbenzimidazole Sulfonic Acid vendu notamment sous le nom commercial EUSOLEX 232 par MERCK
- Benzimidazilate vendu sous le nom commercial NEO HELIOPAN AP par HAARMANN ET REIMER,

### dérivés de triazine :

- Anisotriazine vendue sous le nom commercial TINOSORB S par CIBA GEIGY
- Ethylhexyl triazone vendue notamment sous le nom commercial UVINUL T150 par BASF,
- Diéthylhexyl Butamido Triazone vendue sous le nom commercial UVASORB HEB par SIGMA 3V,

### dérivés de phénylbenzotriazole :

- Drometrizole Trisiloxane vendu sous le nom commercial SILATRIZOLE par RHODIA CHIMIE,

### dérivés anthraniliques :

a) Menthyl Anthranilate vendu sous le nom commercial NEO HELIOPAN MA par HAARMAN ET REIMER,

### dérivés d'imidazolines :

b) Ethylhexyl Dimethoxybenzylidene Dioxoimidazoline Propionate

### dérivés du benzalmalonate :

c) Polyorganosiloxane à fonctions benzalmalonate vendu sous le nom commercial PARSOL SLX par HOFFMANN LAROCHE

Les filtres UV organiques hydrosolubles ou liposolubles plus particulièrement préférés sont choisis parmi les composés suivants :
Ethylhexyl Salicylate,
Butyl Methoxydibenzoylmethane,
Ethylhexyl Methoxycinnamate,
Octocrylene,
Phenylbenzimidazole Sulfonic Acid,
Terephthalylidene Dicamphor Sulfonic Acid,
Benzophenone-3,
Benzophenone-4,
Benzophenone-5,
Benzimidazilate,
Anisotriazine,
Ethylhexyl Triazone,
Diethylhexyl Butamido Triazone,
Drometrizole Trisiloxane,
et leurs mélanges.

Les compositions cosmétiques selon l'invention peuvent contenir en outre des agents de bronzage et/ou de brunissage artificiel de la peau (agents autobronzants) tels que la dihydroxyacétone (DHA).
Les compositions cosmétiques selon l'invention peuvent contenir en outre un ou plusieurs pigments minéraux et en particulier des nanopigments d'oxydes métalliques, enrobés ou non, comme par exemple les nanopigments d'oxyde de titane sous forme amorphe ou cristallisée (rutile et/ou anatase), d'oxyde de fer, d'oxyde de zinc, d'oxyde de zirconium ou d'oxyde de cérium. Ces nanopigments ont une taille moyenne de particules comprise entre 5 nm et 100 nm, de préférence entre 10 nm et 50 nm et sont tous des agents photoprotecteurs UV connus.
Ces nanopigments peuvent être enrobés par des agents d'enrobage connus comme par exemple l'alumine et/ou le stéarate d'aluminium.
De tels nanopigments enrobés ou non enrobés sont décrits par exemple dans les demandes de brevets EP-A-0 518 772 et EP-A-0 518 773.
Les compositions cosmétiques peuvent contenir en outre des adjuvants de formulation usuels tels que les corps gras, les solvants organiques physiologiquement acceptables, les silicones, les agents tensioactifs, les polymères anioniques, cationiques, non ioniques, amphotères ou leurs mélanges, les agents épaississants, les agents antioxydants, les agents opacifiants, les agents stabilisants, les agents anti-mousse, les parfums, les agents conservateurs, les charges, les agents séquestrants, les agents propulseurs, les agents d'ajustement du pH, les colorants et leurs mélanges.

Elles peuvent contenir en outre un ou plusieurs principes actifs cosmétiques choisis par exemple parmi les agents adoucissants, les hydroxyacides, les vitamines, les agents hydratants, les agents émollients, les agents anti-radicalaires, les antagonistes de substance P, les agents anti-inflammatoires et des mélanges de ces composés.
Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les polyoléfines, les huiles fluorées et perfluorées. De même, les cires peuvent être choisies parmi les cires animales, fossiles, végétales, minérales ou de synthèse connues en soi.
Parmi les solvants organiques, on peut citer les alcools et polyols inférieurs tels que l'éthanol, l'isopropanol, le propylèneglycol, la glycérine et le sorbitol.
Les épaississants peuvent être choisis notamment parmi les gommes de guar et celluloses, modifiées ou non, telles que la gomme de guar hydroxypropylée, la méthylhydroxyéthylcellulose, l'hydroxypropylméthylcellulose ou encore l'hydroxyéthylcellulose.
Bien entendu l'homme du métier veillera à choisir ce ou ces éventuels composés complémentaires et/ou leurs quantités de manière à ce que les propriétés avantageuses intrinsèques à l'invention, et en particulier la stabilité des dispersions, ne soient pas altérées par la ou les adjonctions envisagées.
Les compositions de l'invention peuvent être préparées selon les techniques bien connues de l'homme du métier, en particulier celles destinées à la préparation d'émulsions de type huile-dans-eau ou eau-dans-huile.
Cette composition peut se présenter en particulier sous forme d'émulsion, simple ou complexe (H/E, E/H, H/E/H ou E/H/E) telle qu'une crème, un lait, un gel ou un gel-crème, et peuvent éventuellement être conditionnée en aérosol et se présenter sous forme de mousse ou de spray.
Lorsqu'il s'agit d'une émulsion, la phase aqueuse de celle-ci peut comprendre une dispersion vésiculaire non ionique préparée selon des procédés connus (Bangham, Standish and Watkins, *J. Mol. Biol.* 13, 238 (1965), FR 2 315 991 et FR 2 416 008).
La composition cosmétique de l'invention peut être utilisée comme composition protectrice de l'épiderme humain ou des cheveux contre les rayons ultraviolets, comme composition anti-solaire ou comme produit de maquillage.
Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de mousse aérosol ou de spray.
Lorsque la composition cosmétique selon l'invention est utilisée pour la protection des cheveux contre les rayons UV, elle peut se présenter sous forme de shampooing, de lotion, de gel, d'émulsion, de dispersion vésiculaire non ionique et constituer par exemple une composition à rincer, à appliquer avant ou après shampoooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou de décoloration des cheveux.
Lorsque la composition est utilisée comme produit de maquillage des cils, des sourcils ou de la peau, tel que crème de traitement de l'épiderme, fond de tein, bâton de rouge à lèvres, fard à paupières, fard à joues, mascara ou ligneur encore appelé "eye-liner", elle peut se présenter sous forme aqueuse, comme des émulsions huile-dans-eau ou eau-dans-huile, des dispersions vésiculaires non ioniques ou encore des suspensions.
A titre indicatif, pour les formulations anti-solaires conformes à l'invention, qui présentent un support de type émulsion huile-dans-eau, la phase aqueuse représente généralement de 50 à 95 % en poids, de préférence de 70 à 90 % en poids, par rapport à l'ensemble de la formulation, la phase huileuse de 5 à 50 % en poids, de préférence de 10 à 30 % en poids, par rapport à l'ensemble de la formulation, et le ou les (co)émulsionnant(s) de 0,5 à 20 % en poids, de préférence de 2 à 10 % en poids, par rapport à l'ensemble de la formulation.

## Revendications

1. Utilisation d'un copolymère amphiphile comportant au moins une séquence hydrophile et au moins une séquence hydrophobe pour stabiliser des dispersions de composés organiques insolubles comportant au moins un groupe absorbant le rayonnement UV se présentant sous forme de particules ayant une taille moyenne comprise entre 10 nm et 5 µm.

2. Utilisation selon la revendication 1, **caractérisée par le fait que** les dispersions de composés organiques insolubles comportant au moins un groupe absorbant le rayonnement UV sont des dispersions aqueuses.

3. Utilisation selon la revendication 1 ou 2, **caractérisée par le fait que** le copolymère amphiphile comportant au moins une séquence hydrophile et au moins une séquence hydrophobe est un polymère non ionique.

4. Utilisation selon la revendication 3, **caractérisée par le fait que** le copolymère amphiphile comportant au moins une séquence hydrophile et au moins une séquence hydrophobe est choisi dans le groupe formé par
• les alcools aliphatiques polyalcoxylés de formule :
H₂ₙ₊₁Cₙ(CH₂CH₂O)ₓ(CH₂CH(CH₃)O)_{y}(CH(CH₃)CH₂O)_{z}H
dans laquelle
n est un nombre entier compris entre 5 et 20, de préférence entre 7 et 19,
x est un nombre entier compris entre 2 et 20, de préférence entre 4 et 10,
y est un nombre entier compris entre 2 et 20, de préférence entre 4 et 10,
la somme de y + z étant un nombre entier compris entre 2 et 20, de préférence entre 2 et 12 ;
• les copolymères triséquencés d'oxyde d'éthylène et d'oxyde de propylène, de formule
HO(CH₂CH₂O)ₐ(CH₂CH(CH₃)O)_{b}(CH₂CH₂O)_{c}H
dans laquelle a représente un nombre entier compris entre 2 et 150, b représente un nombre entier compris entre 10 et 80 et c représente un nombre entier compris 2 et 150 ;
• les produits de condensation de copolymères séquencés d'oxyde d'éthylène et d'oxyde de propylène sur l'éthylènediamine correspondant à la formule : 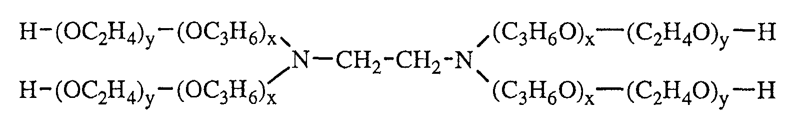
• les copolymères séquencés de styrène et d'oxyde d'éthylène,
• les polymères diuréthanne d'alcools gras polyalcoxylés de formule : 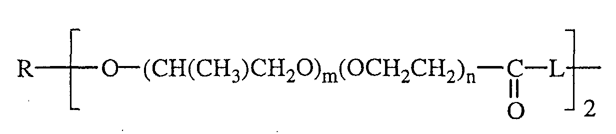 dans laquelle
n représente un nombre compris entre 40 et 70, m représente un nombre compris entre 5 et 20, R représente un groupe alkyle en C₈₋₃₀ et L représente un groupe dérivé d'isophorone-diisocyanate ou d'hexaméthylène-diisocyanate,
• les polyoléfine-succinates polyéthoxylés, et en particulier le polyisobutylène-succinate polyéthoxylé de formule 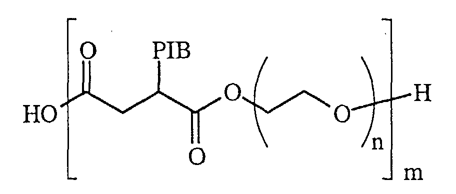 dans laquelle
PIB représente une chaîne de polyisobutylène, n représente un nombre entier compris entre 2 et 15, de préférence entre 7 et 10, et m représente un nombre entier compris entre 2 et 15, de préférence entre 2 et 6 ;
• les terpolymères séquencés d'acide (méth)acrylique, de (méth)acrylate d'alkyle en C₁₀₋₃₀ et de (méth)acrylate de polyéthylèneglycol.

5. Utilisation selon l'une quelconque des revendications précédentes, **caractérisée par le fait que** les composés organiques insolubles comportant au moins un groupe absorbant le rayonnement UV sont choisis parmi les filtres UV insolubles de type oxalanilide, triazine, benzotriazole, amide vinylique, cinnamide, benzazole, benzofuranne, arylvinylidène-cétone, amide d'acrylonitrile, sulfonamide d'acrylonitrile, carbamate d'acrylonitrile ou phénylène-bis-benzoxazinone.

6. Utilisation selon la revendication 5, **caractérisée par le fait que** les filtres UV insolubles de type oxalanilide sont choisis parmi ceux de formule 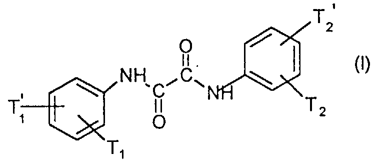 dans laquelle T₁, T₁', T₂ et T₂', représentent chacun indépendamment un radical alkyle en C₁₋₈ ou un radical alcoxy en C₁₋₈.

7. Utilisation selon la revendication 5, **caractérisée par le fait que** les filtres UV insolubles de type triazine sont choisis parmi ceux de formule 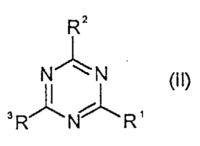 dans laquelle R₁, R₂ et R₃ représentent chacun indépendamment un groupe phenyle, phenoxy ou pyrrolo non substitués ou portant chacun indépendamment un, deux ou trois substituants choisis parmi -OH, alkyle en C₁₋₁₈, alkoxy en C₁₋₁₈, carboxy(alkyle en C₁₋₁₈), cycloalkyle en C₅-C₈, méthylbenzylidène-camphre, -(CH=CR')ₙ(CO)-OR₄ où R' représente un atome d'hydrogène, un groupe cyano ou un groupe COOR₄, avec R₄ = alkyle en C₁₋₁₈ ou cinnamyle, et n vaut 0 ou 1.

8. Utilisation selon la revendication 5, **caractérisée par le fait que** les filtres UV insolubles de type benzotriazole sont choisis parmi ceux de formule 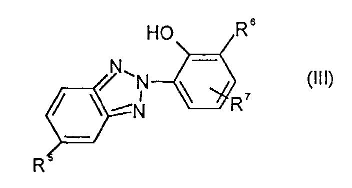 dans laquelle R₅ désigne un atome d'hydrogène ou un radical alkyle en C₁₋₁₈, R₆ et R₇, identiques ou différents, désignent un radical alkyle en C₁₋₁₈ éventuellement substitué par un groupe phényle,
ou de formule 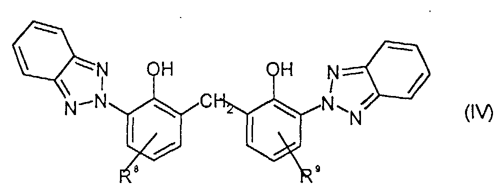 dans laquelle R₈ et R₉, identiques ou différents, représentent chacun un radical alkyle en C₁₋₁₈ pouvant être substitué par un ou plusieurs radicaux choisis parmi alkyle en C₁₋₄, cycloalkyle en C₅₋₁₂ ou aryle.

9. Utilisation selon la revendication 5, **caractérisée par le fait que** les filtres UV insolubles de type amide vinylique sont choisis parmi ceux de formule
T₃-(Y)ᵣ-C(=O)-C(T₄)=C(T₅)-N(T₆)(T₇) (V)
dans laquelle T₃ est un radical alkyle en C₁₋₁₈, de préférence en C₁₋₅, ou un groupe phényle éventuellement substitué par un, deux ou trois radicaux choisis parmi OH, alkyle en C₁₋₁₈, alcoxy en C₁₋₈ ou -C(=O)-OT₈ où T₈ représente un groupe alkyle en C₁₋₁₈ ; T₄, T₅, T₆ et T₇ représentent chacun indépendamment un radical alkyle en C₁₋₁₈, de préférence en C₁₋₅, ou un atome d'hydrogène ; Y représente -NH- ou un atome d'oxygène et r vaut 0 ou 1.

10. Utilisation selon la revendication 5, **caractérisée par le fait que** les filtres UV insolubles de type cinnamide sont choisis parmi ceux de formule 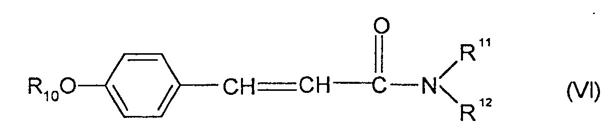 dans laquelle
R₁₀ représente un atome d'hydrogène ou un groupe alkyle en C₁₋₄,
R₁₁ représente un atome d'hydrogène ou un groupe alkyle en C₁₋₄,
R₁₂ représente un groupe -(CONH)ₛ-phényle où s vaut 0 ou 1 et le groupe phényle peut être substitué par un, deux ou trois groupes choisis parmi OH, alkyle en C₁₋₁₈, alcoxy en C₁₋₈ ou -C(=O)-OR₁₃ où R₁₃ est un alkyle en C₁₋₁₈, et plus préférentiellement R₁₂ représente un groupe phényle, 4-méthoxyphényle ou phénylaminocarbonyle.

11. Utilisation selon la revendication 5, **caractérisée par le fait que** les filtres insolubles de type cinnamide sont des bis-(cinnamides α,β disubstitués) choisis parmi ceux de formule : 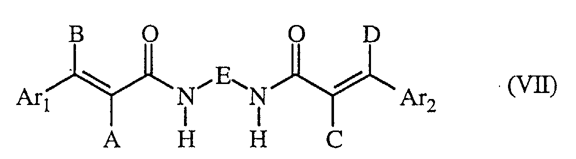 dans laquelle
Ar₁ et Ar₂, identiques ou différents, représentent chacun un radical phényle, un hétérocycle aromatique, un groupe à noyau phényle condensé ou un groupe à hétérocycle aromatique condensé, et peuvent porter un ou plusieurs substituants, identiques ou différents,
B et D, différents d'un atome d'hydrogène, représentent chacun indépendamment un radical organique,
A et C représentent chacun indépendamment un radical organique, et
E représente un radical organique divalent,
à l'exclusion des composés pour lesquels Ar₁ et Ar₂ représentent tous les deux un groupe phényle portant un substituant -OR où R représente un atome d'hydrogène ou un radical organique, A et C représentent tous les deux un groupe cyano, B et D représentent tous les deux un groupe alkyle ou alcényle en C₁₋₃₅, et E représente un radical organique divalent.

12. Utilisation selon la revendication 5, **caractérisée par le fait que** les filtres UV insolubles de type benzazole ou benzofurane sont choisis parmi ceux de formule 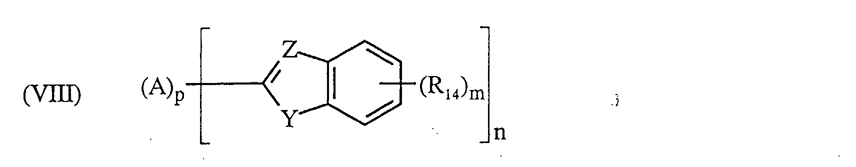 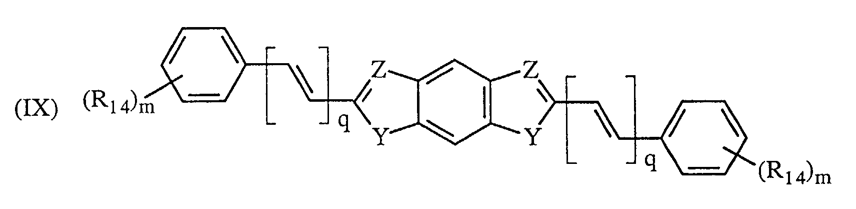 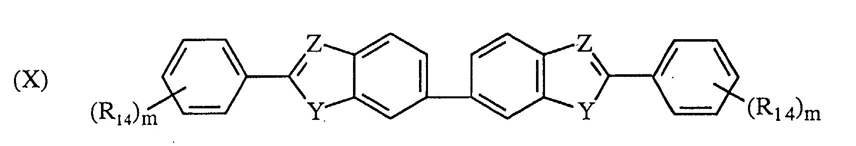 dans lesquelles
chacun des symboles Y représente indépendamment un atome d'oxygène ou de soufre ou un groupe NR₁₅,
chacun des symboles Z représente indépendamment un atome d'azote ou un groupe CH,
chacun des symboles R₁₄ représente indépendamment un groupe OH, un atome d'halogène, un groupe alkyle en C₁₋₈, linéaire ou ramifié, contenant éventuellement un atome de silicium, ou un groupe alcoxy en C₁₋₈, linéaire ou ramifié,
chacun des nombres m vaut indépendamment 0, 1 ou 2,
n représente un nombre entier compris entre 1 et 4 inclus,
p est égal à 0 ou 1,
chacun des nombres q est égal indépendamment à 0 ou 1,
chacun des symboles R₁₅ représente indépendamment un atome d'hydrogène, un groupe benzyle ou alkyle en C₁₋₈, linéaire ou ramifié, contenant éventuellement un atome de silicium,
A représente un radical de valence n choisi parmi ceux de formules : 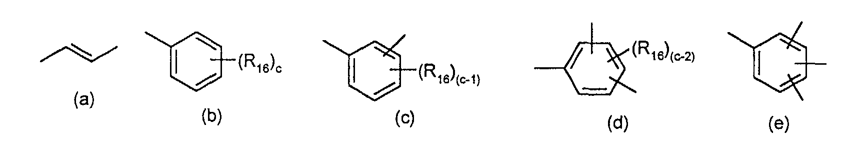 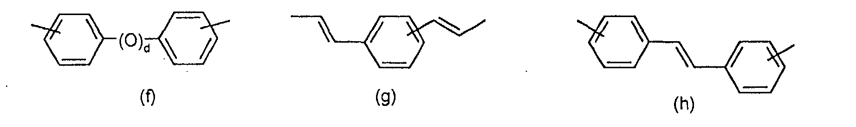 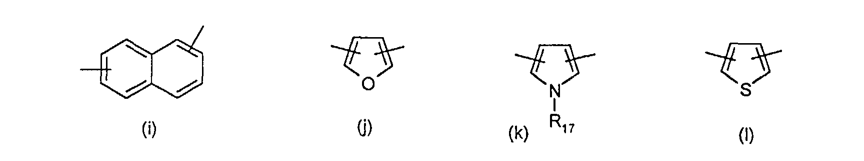 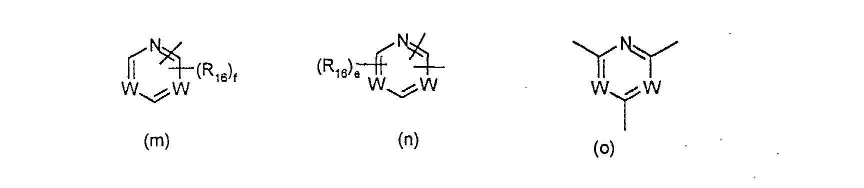 dans lesquelles chacun des symboles R₁₆ représente indépendamment un atome d'halogène ou un groupe alkyle ou alcoxy en C₁₋₄, linéaire ou ramifié, ou hydroxy,
R₁₇ représente un atome d'hydrogène ou un groupe alkyle en C₁₋₄, linéaire ou ramifié,
c = 0 - 4, d = 0 - 3, e = 0 ou 1 et f = 0 - 2.

13. Utilisation selon la revendication 5, **caractérisée par le fait que** les filtres insolubles de type arylvinylène-cétone sont choisis parmi ceux de formule : 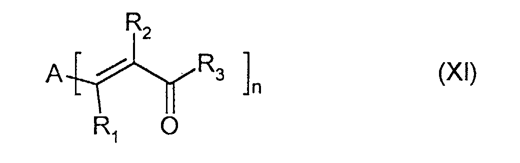 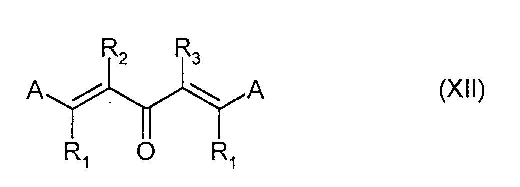 dans lesquelles :
n = 1 ou 2,
A, dans la formule (XI) lorsque n = 1 ou dans la formule (XII), est un radical aryle choisi parmi les formules (a) à (d) suivantes, ou dans la formule (XI) lorsque n=2, est un radical choisi parmi les formules (e) à (h) suivantes : 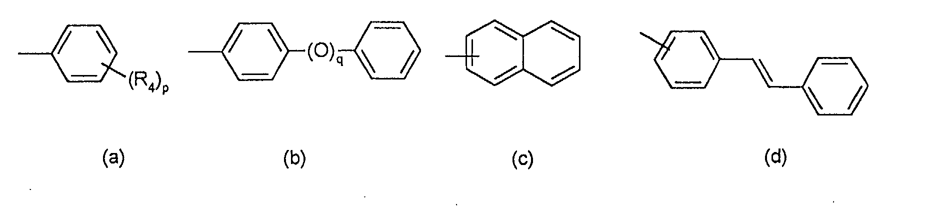 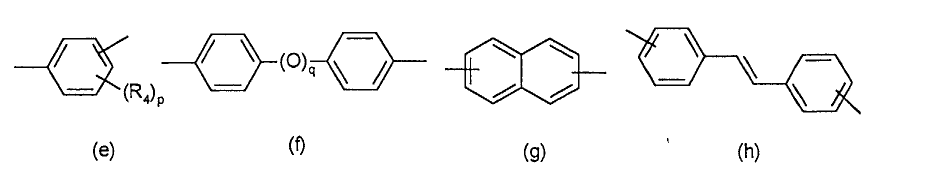
dans lesquelles :
chacun des symboles R₄ représente indépendamment un groupe OH, un atome d'halogène, un groupe alkyle en C₁₋₆, linéaire ou ramifié et contenant éventuellement un atome de silicium, un groupe alcoxy en C₁₋₆, linéaire ou ramifié et contenant éventuellement un atome de silicium, un groupe alcoxycarbonyle en C₁₋₅, linéaire ou ramifié, ou un groupe alkylsulfonamide en C₁₋₆, linéaire ou ramifié et contenant éventuellement un atome de silicium ou une fonction aminoacide,
p représente un nombre entier compris entre 0 et 4 inclus,
q représente 0 ou 1,
R₁ représente l'hydrogène ou un groupe OH,
R₂ représente l'hydrogène, un groupe alkyle en C₁₋₆, linéaire ou ramifié et contenant éventuellement un atome de silicium, un groupe cyano, un groupe alkylsulfonyle en C₁₋₆, un groupe phénylsulfonyle,
R₃ représente un groupe alkyle en C₁₋₆, linéaire ou ramifié et contenant éventuellement un atome de silicium ou un groupe phényle pouvant former un bicycle et éventuellement substitué par un ou deux radicaux R₄,
ou R₂ et R₃ forment ensemble un reste hydrocarboné en C₂₋₁₀ monocyclique, bicyclique ou tricylique, éventuellement interrompu par un ou des atomes d'azote, de soufre et d'oxygène et pouvant contenir un autre carbonyle, et éventuellement substitué par un groupe alkylsulfonamide en C₁-C₈, linéaire ou ramifié, et contenant éventuellement un atome de silicium ou une fonction aminoacide ; à condition que lorsque n=1, R₂ et R₃ ne forment pas un noyau camphre.

14. Utilisation selon la revendication 5, **caractérisée par le fait que** les filtres insolubles de type amides d'acrylonitrile, sulfonamides d'acrylonitrile et carbamates d'acrylonitrile sont choisis parmi ceux de formule : 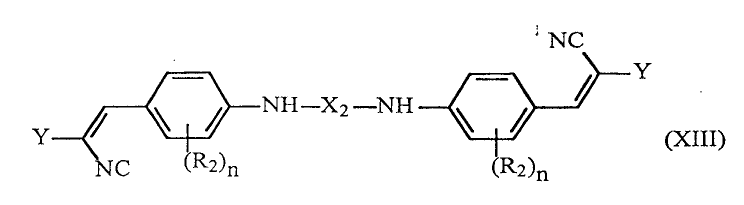 dans laquelle
X₂ représente un radical divalent de formule -(C=O)-R'₃-(C=O)-,
-SO₂-R"₃-SO₂- ou -(C=O)-O-R"₃-O-(C=O)-,
Y représente un radical -(C=O)-R₄ ou -SO₂R₅,
R₂ représente un groupe alkyle en C₁₋₈, linéaire ou ramifié,
n vaut 0, 1 ou 2,
R'₃ représente une simple liaison ou R"₃,
R"₃ représente un radical divalent alkylène en C₁₋₃₀ ou alcénylène en C₃₋₃₀, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium,.
R₄ représente un radical -OR₆ ou -NHR₆,
R₅ représente un radical alkyle en C₁₋₃₀, linéaire ou ramifié, ou un noyau phényle pouvant être substitué par des radicaux alkyle ou alcoxy en C₁₋₄,
R₆ représente un radical alkyle en C₁₋₃₀ ou alcényle en C₃₋₃₀, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium.

15. Utilisation selon la revendication 5, **caractérisée par le fait que** les filtres organiques insolubles de type phénylène-bis-benzoxazinone sont choisis parmi ceux de formule : 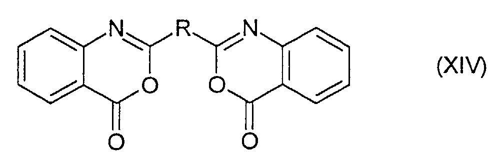 où R représente un reste aromatique divalent choisi parmi les formules (e) à (h) suivantes : 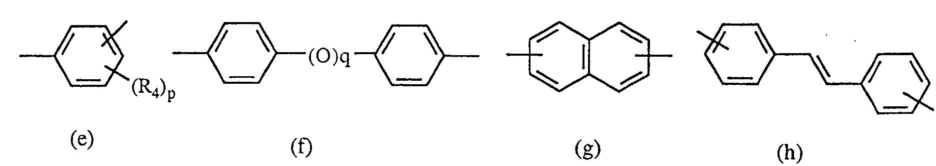 dans lesquelles :
chacun des symboles R₄ représente indépendamment un groupe OH, un atome d'halogène, un groupe alkyle en C₁₋₆, linéaire ou ramifié et contenant éventuellement un atome de silicium, un groupe alcoxy en C₁₋₆, linéaire ou ramifié et contenant éventuellement un atome de silicium, un groupe alcoxycarbonyle en C₁₋₅, linéaire ou ramifié, ou un groupe alkylsulfonamide en C₁₋₆, linéaire ou ramifié et contenant éventuellement un atome de silicium ou une fonction aminoacide,
p représente un nombre entier compris entre 0 et 4 inclus,
q représente 0 ou 1.

16. Utilisation selon l'une quelconque des revendications précédentes, **caractérisée par le fait que** la taille moyenne des particules de composé organique insoluble comportant au moins un groupe absorbant le rayonnement UV est comprise entre 10 nm et 2 µm, de préférence entre 20 nm et 1,5 µm, et en particulier entre 30 nm et 1,0 µm.

17. Utilisation selon l'une quelconque des revendications précédentes, **caractérisée par le fait que** la dispersion contient de 5 à 70 %, de préférence de 30 à 50 % en poids de particules de composé organique insoluble comportant au moins un groupe absorbant le rayonnement UV.

18. Utilisation selon l'une quelconque des revendications précédentes, **caractérisée par le fait que** le copolymère amphiphile comportant au moins une séquence hydrophile et au moins une séquence hydrophobe est utilisé à raison de 1 à 10 mg/m², de préférence à raison de 1 à 5 mg/m², de surface de particules de composé organique insoluble comportant au moins un groupe absorbant le rayonnement UV.

19. Procédé de préparation d'une dispersion d'un composé organique insoluble comportant au moins un groupe absorbant le rayonnement UV, consistant à soumettre des particules dudit composé organique, en suspension dans un milieu liquide, à une étape de réduction de taille des particules jusqu'à obtention d'une taille moyenne de particules comprise entre 10 nm et 5 µm, et à ajouter avant, pendant ou après l'étape de réduction de taille des particules, au moins un copolymère amphiphile comportant au moins une séquence hydrophile et au moins une séquence hydrophobe.

20. Procédé selon la revendication 19, **caractérisé par le fait que** l'étape de réduction de taille des particules consiste en un broyage dans un milieu aqueux.

21. Procédé selon la revendication 20, **caractérisé par le fait que** l'on ajoute le copolymère amphiphile comportant au moins une séquence hydrophile et au moins une séquence hydrophobe avant ou pendant l'étape de broyage.

22. Procédé selon l'une des revendications 19 à 21, **caractérisé par le fait que** le copolymère amphiphile comportant au moins une séquence hydrophile et au moins une séquence hydrophobe est un polymère non ionique tel que défini dans les revendications 4 et 5.

23. Procédé selon l'une des revendications 19 à 22, **caractérisé par le fait que** le composé organique insoluble comportant au moins un groupe absorbant le rayonnement UV est choisi parmi les filtres UV insolubles de type oxalanilide, triazine, benzotriazole, amide vinylique, cinnamide, benzazole, benzofuranne, arylvinylidène-cétone, amide d'acrylonitrile, sulfonamide d'acrylonitrile, carbamate d'acrylonitrile ou phénylène-bis-benzoxazinone.

24. Procédé selon la revendication 23, **caractérisé par le fait que** les filtres UV insolubles sont choisis parmi ceux décrits dans les revendications 7 à 16.

25. Procédé selon l'une des revendications 19 à 24, **caractérisé par le fait que** les particules de composé organique insoluble comportant au moins un groupe filtrant le rayonnement UV représentent de 5 à 70 % en poids, de préférence de 30 à 50 % en poids de la dispersion finale.

26. Procédé selon l'une des revendications 19 à 25, **caractérisé par le fait que** l'on ajoute le copolymère amphiphile comportant au moins une séquence hydrophile et au moins une séquence hydrophobe à raison de 1 à 10 mg, de préférence à raison de 1 à 5 mg, par m² de surface de particules de composé organique insoluble comportant au moins un groupe filtrant le rayonnement UV après réduction de taille.

27. Dispersion comprenant, dans un milieu liquide,
(a) au moins un composé organique insoluble comportant au moins un groupe filtrant le rayonnement UV sous forme de particules ayant une taille moyenne comprise entre 10 nm et 5 µm, et
(b) en tant qu'agent dispersant, au moins un copolymère amphiphile comportant au moins une séquence hydrophile et au moins une séquence hydrophobe, choisi dans le groupe formé par
• les alcools aliphatiques polyalcoxylés de formule :
H₂ₙ₊₁Cₙ(CH₂CH₂O)ₓ(CH₂CH(CH₃)O)_{y}(CH(CH₃)CH₂O)_{z}H
dans laquelle
n est un nombre entier compris entre 5 et 20, de préférence entre 7 et 19,
x est un nombre entier compris entre 2 et 20, de préférence entre 4 et 10,
y est un nombre entier compris entre 2 et 20, de préférence entre 4 et 10,
la somme de y + z étant un nombre entier compris entre 2 et 20, de préférence entre 2 et 12 ;
• les copolymères triséquencés d'oxyde d'éthylène et d'oxyde de propylène, de formule
HO(CH₂CH₂O)ₐ(CH₂CH(CH₃)O)_{b}(CH₂CH₂O)_{c}H
dans laquelle a représente un nombre entier compris entre 2 et 150, b représente un nombre entier compris entre 10 et 80 et c représente un nombre entier compris 2 et 150 ;
• les produits de condensation de copolymères séquencés d'oxyde d'éthylène et d'oxyde de propylène sur l'éthylènediamine correspondant à la formule : 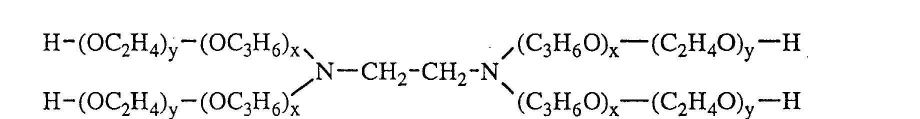
• les copolymères séquencés de styrène et d'oxyde d'éthylène,
• les polyoléfine-succinates polyéthoxylés, et en particulier le polyisobutylène-succinate polyéthoxylé de formule 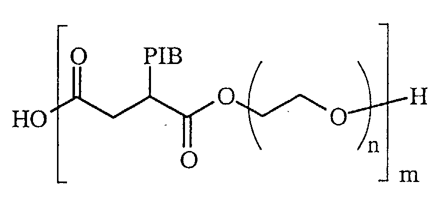 dans laquelle
PIB représente une chaîne de polyisobutylène, n représente un nombre entier compris entre 2 et 15, de préférence entre 7 et 10, et m représente un nombre entier compris entre 2 et 15, de préférence entre 2 et 6 ;
• les terpolymères séquencés d'acide (méth)acrylique, de (méth)acrylate d'alkyle en C₁₀₋₃₀ et de (méth)acrylate de polyéthylèneglycol.

28. Dispersion selon la revendication 27, **caractérisée par le fait qu'**elle contient de 5 à 70 % en poids, de préférence de 30 à 50 % en poids, de particules de composé organique insoluble comportant au moins un groupe filtrant le rayonnement UV.

29. Dispersion selon la revendication 27 ou 28, **caractérisée par le fait qu'**elle contient de 1 à 10 mg, de préférence de 1 à 5 mg, par m² de surface de particules de composé organique insoluble comportant au moins un groupe absorbant le rayonnement UV, d'agent dispersant.

30. Dispersion selon l'une quelconque des revendications 27 à 29, **caractérisée par le fait que** le milieu liquide est un milieu aqueux.

31. Dispersion selon l'une quelconque des revendications 27 à 30, **caractérisée par le fait que** le copolymère amphiphile comportant au moins une séquence hydrophile et au moins une séquence hydrophobe est un polymère non ionique.

32. Dispersion selon l'une quelconque des revendications 27 à 31, **caractérisée par le fait que** les composés organiques insolubles comportant au moins un groupe absorbant le rayonnement UV sont choisis parmi les filtres UV insolubles de type oxalanilide, triazine, benzotriazole, amide vinylique, cinnamide, benzazole, benzofuranne, arylvinylidène-cétone, amide d'acrylonitrile, sulfonamide d'acrylonitrile, carbamate d'acrylonitrile ou phénylène-bis-benzoxazinone.

33. Dispersion selon la revendication 32, **caractérisée par le fait que** les filtres UV insolubles de type oxalanilide sont choisis parmi ceux de formule 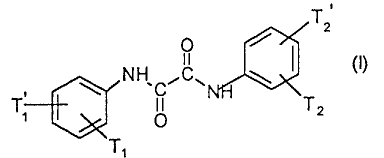 dans laquelle T₁, T₁', T₂ et T₂', représentent chacun indépendamment un radical alkyle en C₁₋₈ ou un radical alcoxy en C₁₋₈.

34. Dispersion selon la revendication 32, **caractérisée par le fait que** les filtres UV insolubles de type triazine sont choisis parmi ceux de formule 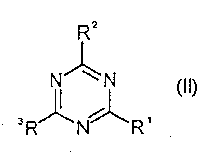 dans laquelle R₁, R₂ et R₃ représentent chacun indépendamment un groupe phenyle, phenoxy ou pyrrolo non substitués ou portant chacun indépendamment un, deux ou trois substituants choisis parmi -OH, alkyle en C₁₋₁₈, alkoxy en C₁₋₁₈, carboxy(alkyle en C₁₋₁₈), cycloalkyle en C₅-C₈, méthylbenzylidène-camphre, -(CH=CR')ₙ(CO)-OR₄ où R' représente un atome d'hydrogène, un groupe cyano ou un groupe COOR₄, avec R₄ = alkyle en C₁₋₁₈ ou cinnamyle, et n vaut 0 ou 1.

35. Dispersion selon la revendication 32, **caractérisée par le fait que** les filtres UV insolubles de type benzotriazole sont choisis parmi ceux de formule 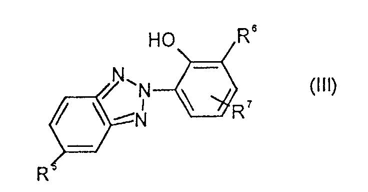 dans laquelle R₅ désigne un atome d'hydrogène ou un radical alkyle en C₁₋₁₈, R₆ et R₇, identiques ou différents, désignent un radical alkyle en C₁₋₁₈ éventuellement substitué par un groupe phényle,
ou de formule 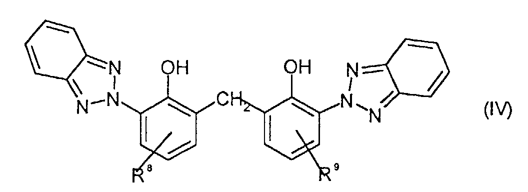 dans laquelle R₈ et R₉, identiques ou différents, représentent chacun un radical alkyle en C₁₋₁₈ pouvant être substitué par un ou plusieurs radicaux choisis parmi alkyle en C₁₋₄, cycloalkyle en C₅₋₁₂ ou aryle.

36. Dispersion selon la revendication 32, **caractérisée par le fait que** les filtres UV insolubles de type amide vinylique sont choisis parmi ceux de formule
T₃-(Y)ᵣ-C(=O)-C(T₄)=C(T₅)-N(T₆)(T₇) (V)
dans laquelle T₃ est un radical alkyle en C₁₋₁₈, de préférence en C₁₋₅, ou un groupe phényle éventuellement substitué par un, deux ou trois radicaux choisis parmi OH, alkyle en C₁₋₁₈, alcoxy en C₁₋₈ ou -C(=O)-OT₈ où T₈ représente un groupe alkyle en C₁₋₁₈ ; T₄, T₅, T₆ et T₇ représentent chacun indépendamment un radical alkyle en C₁₋₁₈, de préférence en C₁₋₅, ou un atome d'hydrogène ; Y représente -NH- ou un atome d'oxygène et r vaut 0 ou 1.

37. Dispersion selon la revendication 32, **caractérisée par le fait que** les filtres UV insolubles de type cinnamide sont choisis parmi ceux de formule 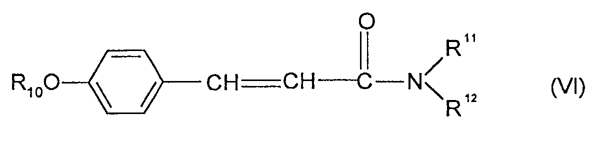 dans laquelle
R₁₀ représente un atome d'hydrogène ou un groupe alkyle en C₁₋₄,
R₁₁ représente un atome d'hydrogène ou un groupe alkyle en C₁₋₄,
R₁₂ représente un groupe -(CONH)ₛ-phényle où s vaut 0 ou 1 et le groupe phényle peut être substitué par un, deux ou trois groupes choisis parmi OH, alkyle en C₁₋₁₈, alcoxy en C₁₋₈ ou -C(=O)-OR₁₃ où R₁₃ est un alkyle en C₁₋₁₈, et plus préférentiellement R₁₂ représente un groupe phényle, 4-méthoxyphényle ou phénylaminocarbonyle.

38. Dispersion selon la revendication 32, **caractérisée par le fait que** les filtres insolubles de type cinnamide sont des bis-(cinnamides α,β disubstitués) choisis parmi ceux de formule : 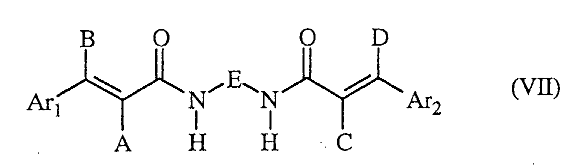 dans laquelle
Ar₁ et Ar₂, identiques ou différents, représentent chacun un radical phényle, un hétérocycle aromatique, un groupe à noyau phényle condensé ou un groupe à hétérocycle aromatique condensé, et peuvent porter un ou plusieurs substituants, identiques ou différents,
B et D, différents d'un atome d'hydrogène, représentent chacun indépendamment un radical organique,
A et C représentent chacun indépendamment un radical organique, et
E représente un radical organique divalent,
à l'exclusion des composés pour lesquels Ar₁ et Ar₂ représentent tous les deux un groupe phényle portant un substituant -OR où R représente un atome d'hydrogène ou un radical organique, A et C représentent tous les deux un groupe cyano, B et D représentent tous les deux un groupe alkyle ou alcényle en C₁₋₃₅, et E représente un radical organique divalent.

39. Dispersion selon la revendication 32, **caractérisée par le fait que** les filtres UV insolubles de type benzazole ou benzofurane sont choisis parmi ceux de formule 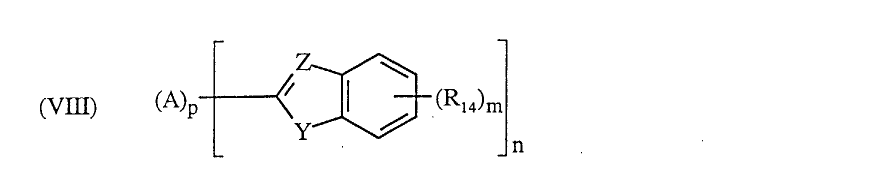 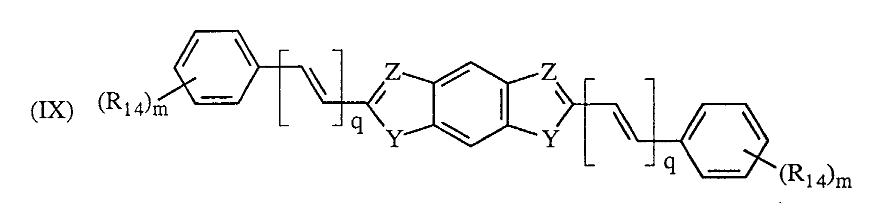 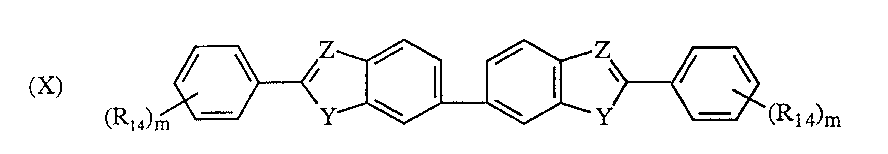 dans lesquelles
chacun des symboles Y représente indépendamment un atome d'oxygène ou de soufre ou un groupe NR₁₅,
chacun des symboles Z représente indépendamment un atome d'azote ou un groupe CH,
chacun des symboles R₁₄ représente indépendamment un groupe OH, un atome d'halogène, un groupe alkyle en C₁₋₈, linéaire ou ramifié, contenant éventuellement un atome de silicium, ou un groupe alcoxy en C₁₋₈, linéaire ou ramifié,
chacun des nombres m vaut indépendamment 0, 1 ou 2,
n représente un nombre entier compris entre 1 et 4 inclus,
p est égal à 0 ou 1,
chacun des nombres q est égal indépendamment à 0 ou 1,
chacun des symboles R₁₅ représente indépendamment un atome d'hydrogène, un groupe benzyle ou alkyle en C₁₋₈, linéaire ou ramifié, contenant éventuellement un atome de silicium,
A représente un radical de valence n choisi parmi ceux de formules : 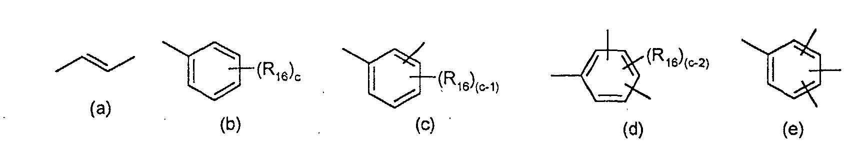 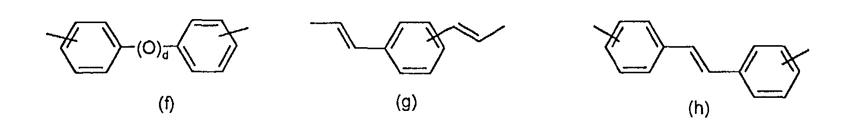 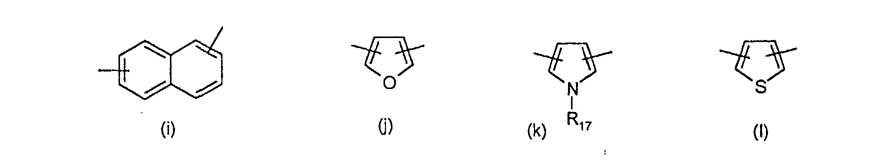 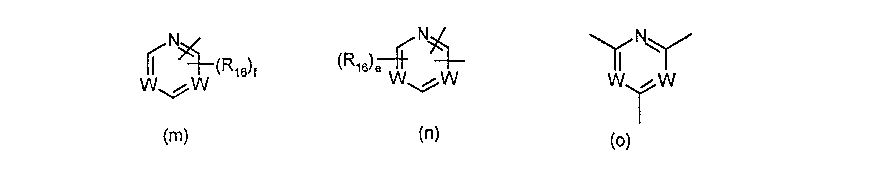 dans lesquelles chacun des symboles R₁₆ représente indépendamment un atome d'halogène ou un groupe alkyle ou alcoxy en C₁₋₄, linéaire ou ramifié, ou hydroxy,
R₁₇ représente un atome d'hydrogène ou un groupe alkyle en C₁₋₄, linéaire ou ramifié,
c = 0 - 4, d = 0 - 3, e = 0 ou 1 et f = 0 - 2.

40. Dispersion selon la revendication 32, **caractérisée par le fait que** les filtres insolubles de type arylvinylène-cétone sont choisis parmi ceux de formule : 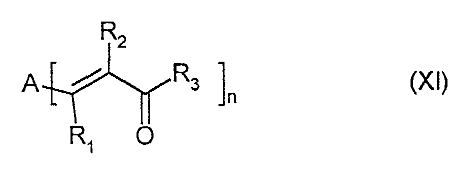 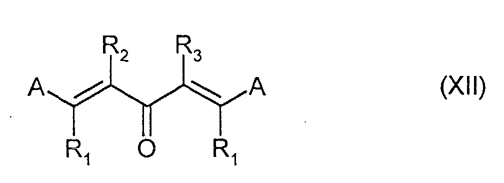 dans lesquelles :
n = 1 ou 2,
A, dans la formule (XI) lorsque n = 1 ou dans la formule (XII), est un radical aryle choisi parmi les formules (a) à (d) suivantes, ou dans la formule (XI) lorsque n=2, est un radical choisi parmi les formules (e) à (h) suivantes : 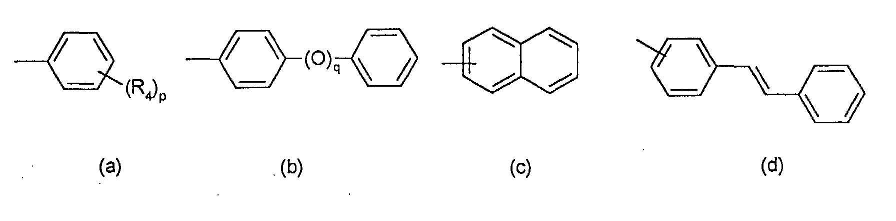 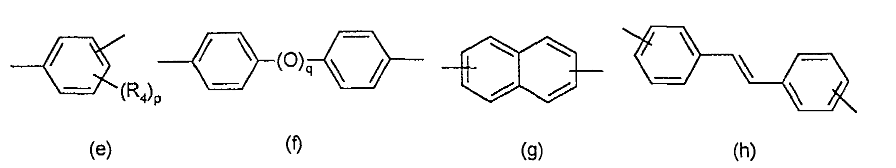
dans lesquelles :
chacun des symboles R₄ représente indépendamment un groupe OH, un atome d'halogène, un groupe alkyle en C₁₋₆, linéaire ou ramifié et contenant éventuellement un atome de silicium, un groupe alcoxy en C₁₋₆, linéaire ou ramifié et contenant éventuellement un atome de silicium, un groupe alcoxycarbonyle en C₁₋₅, linéaire ou ramifié, ou un groupe alkylsulfonamide en C₁₋₆, linéaire ou ramifié et contenant éventuellement un atome de silicium ou une fonction aminoacide,
p représente un nombre entier compris entre 0 et 4 inclus,
q représente 0 ou 1,
R₁ représente l'hydrogène ou un groupe OH,
R₂ représente l'hydrogène, un groupe alkyle en C₁₋₆, linéaire ou ramifié et contenant éventuellement un atome de silicium, un groupe cyano, un groupe alkylsulfonyle en C₁₋₆, un groupe phénylsulfonyle,
R₃ représente un groupe alkyle en C₁₋₆, linéaire ou ramifié et contenant éventuellement un atome de silicium ou un groupe phényle pouvant former un bicycle et éventuellement substitué par un ou deux radicaux R₄,
ou R₂ et R₃ forment ensemble un reste hydrocarboné en C₂₋₁₀ monocyclique, bicyclique ou tricylique, éventuellement interrompu par un ou des atomes d'azote, de soufre et d'oxygène et pouvant contenir un autre carbonyle, et éventuellement substitué par un groupe alkylsulfonamide en C₁-C₈, linéaire ou ramifié, et contenant éventuellement un atome de silicium ou une fonction aminoacide ; à condition que lorsque n=1, R₂ et R₃ ne forment pas un noyau camphre.

41. Dispersion selon la revendication 32, **caractérisée par le fait que** les filtres insolubles de type amides d'acrylonitrile, sulfonamides d'acrylonitrile et carbamates d'acrylonitrile sont choisis parmi ceux de formule : 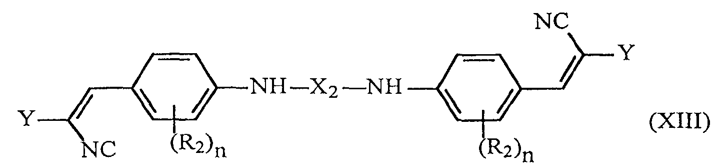 dans laquelle
X₂ représente un radical divalent de formule -(C=O)-R'₃-(C=O)-,
-SO₂-R"₃-SO₂- ou -(C=O)-O-R"₃-O-(C=O)-,
Y représente un radical -(C=O)-R₄ ou -SO₂R₅,
R₂ représente un groupe alkyle en C₁₋₈, linéaire ou ramifié,
n vaut 0, 1 ou 2,
R'₃ représente une simple liaison ou R"₃,
R"₃ représente un radical divalent alkylène en C₁₋₃₀ ou alcénylène en C₃₋₃₀, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium,.
R₄ représente un radical -OR₆ ou -NHR₆,
R₅ représente un radical alkyle en C₁₋₃₀, linéaire ou ramifié, ou un noyau phényle pouvant être substitué par des radicaux alkyle ou alcoxy en C₁₋₄,
R₆ représente un radical alkyle en C₁₋₃₀ ou alcényle en C₃₋₃₀, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium.

42. Dispersion selon la revendication 32, **caractérisée par le fait que** les filtres organiques insolubles de type phénylène-bis-benzoxazinone sont choisis parmi ceux de formule : 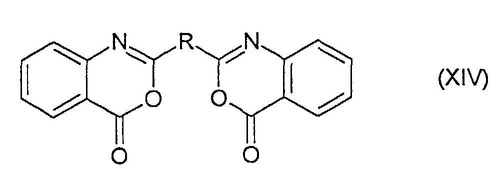 où R représente un reste aromatique divalent choisi parmi les formules (e) à (h) suivantes : 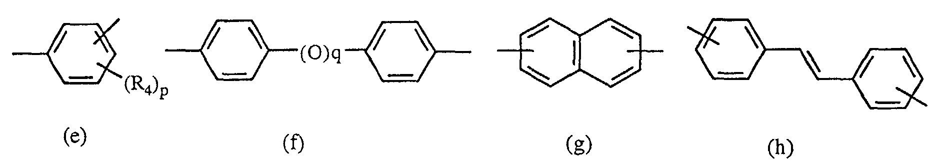 dans lesquelles :
chacun des symboles R₄ représente indépendamment un groupe OH, un atome d'halogène, un groupe alkyle en C₁₋₆, linéaire ou ramifié et contenant éventuellement un atome de silicium, un groupe alcoxy en C₁₋₆, linéaire ou ramifié et contenant éventuellement un atome de silicium, un groupe alcoxycarbonyle en C₁₋₅, linéaire ou ramifié, ou un groupe alkylsulfonamide
en C₁₋₆, linéaire ou ramifié et contenant éventuellement un atome de silicium ou une fonction aminoacide,
p représente un nombre entier compris entre 0 et 4 inclus,
q représente 0 ou 1.

43. Dispersion selon l'une quelconque des revendications 27 à 42, **caractérisée par le fait que** la taille moyenne des particules de composé organique insoluble comportant au moins un groupe absorbant le rayonnement UV est comprise entre 10 nm et 2 µm, de préférence entre 20 nm et 1,5 µm, et en particulier entre 30 nm et 1,0 µm.

44. Composition cosmétique à usage topique, en particulier destinée à la photoprotection de la peau et/ou des cheveux, contenant, dans un milieu cosmétiquement acceptable,
(a) au moins un composé organique insoluble comportant au moins un groupe filtrant le rayonnement UV sous forme de particules ayant une taille moyenne comprise entre 10 nm et 5 µm, et
(b) au moins un agent dispersant choisi parmi les copolymères amphiphiles comportant au moins une séquence hydrophile et au moins une séquence hydrophobe,
préparée par dilution d'une dispersion selon l'une quelconque des revendications 27 à 43, dans un milieu cosmétiquement acceptable.

45. Composition selon la revendication 44, **caractérisée par le fait qu'**elle contient de 0,1 à 15 % en poids, de préférence de 0,2 à 10 % en poids de particules de composé organique insoluble comportant au moins un groupe filtrant le rayonnement UV.

46. Composition selon la revendication 44 ou 45, **caractérisée par le fait qu'**elle contient en outre un ou plusieurs filtres solaires organiques hydrosolubles ou liposolubles actifs dans le domaine des UV-A et/ou UV-B.

47. Composition cosmétique selon l'une des revendications 44 à 46, **caractérisée par le fait que** les filtres solaires organiques solubles sont choisis parmi les suivants : Ethylhexyl Salicylate, Butyl Methoxydibenzoylmethane, Ethylhexyl Methoxycinnamate, Octocrylene, Phenylbenzimidazole Sulfonic Acid, Terephthalylidene Dicamphor Sulfonic Acid, Benzophenone-3, Benzophenone-4, Benzophenone-5, 4-Methylbenzyli-dene Camphor, Benzimidazilate, Aniso-triazine, Ethylhexyl Triazone, Diethylhexyl Butamido Triazone, Methylene bis-Benzotriazolyl Tetramethylbutylphenol, Drometrizole Trisiloxane, et des mélanges de ceux-ci.

48. Composition cosmétique selon l'une des revendications 44 à 47, **caractérisée par le fait qu'**elle contient en outre un ou plusieurs pigments minéraux.

49. Composition cosmétique selon la revendication 48, **caractérisée par le fait que** les pigments minéraux sont choisis parmi les nanopigments d'oxydes métalliques tels que l'oxyde de titane, l'oxyde de fer, l'oxyde de zinc, l'oxyde de zirconium et l'oxyde de cérium, éventuellement enrobés, et leurs mélanges.

50. Composition cosmétique selon la revendication 49, **caractérisée par le fait que** les nanopigments ont une taille moyenne de particules comprise entre 5 nm et 100 nm, de préférence entre 10 nm et 50 nm.

51. Composition cosmétique selon l'une des revendications 44 à 50, **caractérisée par le fait qu'**elle contient en outre au moins un agent de bronzage et/ou de brunissage artificiel de la peau.

52. Composition cosmétique selon l'une quelconque des revendications 44 à 51, **caractérisée par le fait qu'**elle contient en outre un ou plusieurs adjuvants de formulation choisis parmi les corps gras, les solvants organiques physiologiquement acceptables, les agents épaississants, les agents antioxydants, les agents opacifiants, les agents stabilisants, les agents anti-mousse, les parfums, les agents conservateurs, les charges, les agents séquestrants, les agents propulseurs, les agents d'ajustement du pH, les colorants et leurs mélanges.

53. Composition cosmétique selon l'une quelconque des revendications 44 à 52, **caractérisée par le fait qu'**elle contient en outre un ou plusieurs principes actifs cosmétiques choisis parmi les vitamines, les agents adoucissants, les hydroxyacides, les agents hydratants, les agents émollients, les agents anti-radicalaires, les antagonistes de substance P, les agents anti-inflammatoires et des mélanges de ces composés.

54. Composition cosmétique selon l'une quelconque des revendications 44 à 53, **caractérisée par le fait qu'**elle constitue une composition cosmétique destinée à protéger la peau ou les cheveux, une composition anti-solaire ou un produit de maquillage.
